(19) Europäisches Patentamt
European Patent Office
Office européen des brevets

(11) Numéro de publication: 0 463 043 B1

(12) **FASCICULE DE BREVET EUROPEEN**

(45) Date de publication de fascicule du brevet: **18.05.94**

(51) Int. Cl.[5]: **C12Q 1/68**, C07K 7/00, A61K 37/02

(21) Numéro de dépôt: **90904868.8**

(22) Date de dépôt: **13.03.90**

(86) Numéro de dépôt internationale :
**PCT/FR90/00168**

(87) Numéro de publication internationale :
**WO 90/10713 (20.09.90 90/22)**

(54) **PROCEDE DE STABILISATION DE L'HYBRIDATION DE SEOUENCES POLYNUCLEOTIOUES COMPLEMENTAIRES.**

(30) Priorité: **14.03.89 FR 8903303**

(43) Date de publication de la demande:
**02.01.92 Bulletin 92/01**

(45) Mention de la délivrance du brevet:
**18.05.94 Bulletin 94/20**

(84) Etats contractants désignés:
**AT BE CH DE DK ES FR GB IT LI LU NL SE**

(56) Documents cités:

**Chemical abstracts, vol. 108, n 25, 20 juin 1988 (Columbus, Ohio, US), B.V. Tyaglov et al "Nucleoamino acids and peptides XV. Reaction of arginine-containing nucleopeptides with polynucleotides", voir page 615, résumé 222112a & Zh. Obshch. Khim. 1987, 57(9), 2124-6**

(73) Titulaire: **OPALE BIOTECHNOLOGY**
**Athélia 2 Zone Industrielle, La Plaine Brunette**
**F-13600 La Ciotat(FR)**

(72) Inventeur: **PRIEUR, Benoît**
**36, rue Monge**
**F-75005 Paris(FR)**

(74) Mandataire: **Warcoin, Jacques et al**
**Cabinet Régimbeau**
**26, avenue Kléber**
**F-75116 Paris (FR)**

EP 0 463 043 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Chemical abstracts, vol. 106, n 23, 8 juin 1987, (Columbus, Ohio, US) K. Zakrzewska et al "Theoretical studies on the interaction of proteins and nucleic acid II. The binding of alpha-helix to B-DNA" voir page 310, résumé 191492b & Biophys. Chem. 1986, 25(2), 201-13

Proc. Natl. Acad. Sci. USA, vol. 84, février 1987, M. Lemaitre et al "Specific antiviral activity of a poly(L-lysin)-conjugated oligodeoxyribonucleotide sequence complementary to vesicular stomatitis virus N protein mRNA initiation site", voir pages 648-652

## Description

La présente invention concerne un procédé de stabilisation de l'hybridation d'une première séquence polunucléotidique d'ADN ou ARN, c'est-à-dire polyribonucléotidique ou Polydésoxyribonucléotidique, avec une seconde séquence polynucléotidique cible complémentaire de la première.

La présente invention concerne également de nouveaux conjugués utiles pour la mise en oeuvre du procédé selon l'invention consistant dans le couplage d'une séquence polynucléotidique d'ADN ou ARN avec une séquence peptidique.

Enfin, l'invention concerne l'application de ce procédé et de ces nouveaux conjugués dans le domaine du diagnostic à titre de sonde d'acides nucléiques et dans le domaine thérapeutioue à titre d'agent de blocage ou d'inhibition de l'expression de gènes.

L'emploi de séquences polynucléotidiques d'ADN ou ARN faisant intervenir leur capacité d'appariement avec des séquences polynucléotidiques complémentaires à des fins de diagnostic, c'est-à-dire comme sonde lorsque ces séquences sont marquées ou à des fins thérapeutiques comme agent de blocage sélectif de l'expression d'un gène qu'il soit exogène (ADN ou ARN de virus, de parasites ou de bactéries) ou endogène (inhibition de la traduction de l'ARN messager), est maintenant bien connu.

Une difficulté réside toutefois dans la nécessaire stabilisation de l'appariement, c'est-à-dire de l'hybridation entre les deux séquences complémentaires, sans compter les principales difficultés rencontrées lors de l'utilisation de ces molécules qui sont dues principalement à cinq causes :

1) une très faible efficacité de pénétration
2) une faible durée de vie des molécules non protégées
3) une instabilité des dérivés protégés
4) une perte de spécificité au profit de l'affinité de certains dérivés ($\alpha$ anomères)
5) une inhibition de l'expression d'un ARNm, due le plus souvent à une digestion RNasique (lorsque cela est possible) plutôt qu'au blocage du site d'attachement du ribosome.

Pour surmonter ces difficultés, on est contraint d'utiliser des séquences suffisamment longues pour obtenir une bonne spécificité, mais pas trop longues pour éviter l'apparition de structure secondaire due à un repliement interne de la molécule.

On a proposé d'adjoindre au polynucléotide des agents intercalants, tels que l'acridine. Enfin, plus récemment, il a également été proposé d'utiliser des séquences polynucléotidiques dont les nucléotides présentent une configuration anomérique non naturelle $\alpha$. Ces séquences à anomérie $\alpha$ s'apparient de façon parallèle et plus stable avec des séquences complémentaires à anomérie $\beta$ naturelle. En effet, les séquences polynucléotidiques d'anoméries $\beta$ complémentaires s'apparient de façon antiparallèle. Toutefois, il se produit des erreurs d'appariement avec la séquence cible faisant perdre à la molécule sa spécificité.

Le but de la présente invention est donc de stabiliser l'hybridation de séquences complémentaires d'ADN ou ARN tout en palliant aux inconvénients précités de l'état de la technique.

La présente invention a en effet pour objet un procédé de stabilisation de l'hybridation d'une première séquence polynucléotidique d'ADN ou d'ARN modifiés ou non, à configuration anomérique $\alpha$ ou $\beta$, avec une seconde séquence cible complémentaire de la première d'ADN ou d'ARN, caractérisé en ce qu'un peptide forme un complexe avec la double hélice d'hybridation que forment lesdites première et seconde séquences nucléotidiques en prenant une structure en épingle à cheveux dans le grand sillon de ladite double hélice.

Le peptide peut être fixé ou non à ladite première séquence.

Dans un mode de réalisation particulier de l'invention, le peptide est fixé sur ladite première séquence.

Il convient de noter que Lemaître et al. (PNAS) 1987, 84, 648-652 ont couplé un peptide de polylysine à un oligonucléotide, afin d'en augmenter la pénétration, le peptide n'ayant aucun rôle dans la stabilisation de l'hybride. En outre, la polylysine est très toxique pour la cellule.

Dans une première variante de réalisation de l'invention, l'enchaînement d'aminoacides du peptide comporte :

. une première succession d'aminoacides qui présentent une interaction un à un respectivement avec une succession de bases de nucléotides de ladite première séquence nucléotidique, le cas échéant, à partir du site de fixation du peptide sur celle-ci,
. une seconde succession d'un même nombre d'aminoacides que la première succession d'aminoacides, qui présentent une interaction un à un respectivement avec une succession de bases de nucléotides de ladite seconde séquence,
. lesdites première et seconde successions d'aminoacides étant reliées entre elles par une séquence dite "charnière" constituée notamment par un ou plusieurs acides aminés n'interagissant pas avec les bases desdites première et seconde séquences,

de telle sorte que

. les acides aminés de ladite première succession interagissent de manière coopérative avec les acides aminés de ladite seconde succession d'acides aminés,
. l'interaction coopérative résultante entre le peptide et l'hélice double brin formée par le complexe d'hybridation desdites première et seconde séquences étant telle que le peptide se glisse dans le grand sillon de la double hélice et prenne une structure en épingle à cheveux entre ladite première et ladite seconde séquences nucléotidiques par l'intermédiaire de la séquence dite "charnière".

La présente invention découle des résultats d'un travail effectué par le demandeur sur l'origine du code génetique.

Selon l'invention, le peptide prend une structure en épingle à cheveux et se glisse dans le grand sillon de l'hélice nucléotidique double brin en prenant la configuration telle que représentée sur les figures 1 à 3 dans lesquelles :

. chaque azote d'une liaison peptidique est à l'origine de deux liaisons hydrogène (excepté la Proline); une comme accepteur d'hydrogène appelée E (Electron) et une autre comme donneur d'hydrogène appelée H. L'azote peptidique a une structure tétraédrique dans ce modèle.

Il est convenu que E (Electron) interagit avec le $NH_2$ en C4 de la cytosine ou le $NH_2$ en C6 de l'adénine entre autres bases, et que H interagit avec l'O4 de la thymine ou de l'uracyle ou de l'O6 de la guanine entre autres bases. Le site E ou H resté libre interagit avec celui de la partie peptidique antiparallèle resté également libre.

Le radical R de l'acide aminé de série L de formule NH-HCR-CO interagit avec le reste de la surface exposée au grand sillon de la base $B_2$, du côté amide de l'acide aminé.

Dans ce type d'interaction coopérative, un mauvais appariement des nucléotides, par exemple G-T, C-A, G-A ou T-C, empêche une stabilisation globale du complexe.

L'interaction coopérative acides aminés-acides aminés entre les deux branches de l'épingle à cheveux peptidique est à l'origine selon la présente invention du processus de stabilisation de l'hybridation entre lesdites première et seconde séquences.

On notera que la forte interaction entre le peptide et la double hélice peut faire apparaître, dans certaines conditions de pH, de force ionique et en présence de certains sels, une double activité de nature protéasique et nucléasique. En effet, si la cible est un RNA, il est possible que l'OH en position 2' du ribose attaque le carbone $\delta +$ du carbonyl peptidique. De cette attaque résulte alors un clivage du RNA cible et du peptide. Ce type d'action est particulièrement intéressant dans le cadre d'une application comme agents thérapeutiques du conjugué Peptide-polynucléotides.

Dans la première variante de réalisation évoquée précédemment, le peptide peut être relié par une seule liaison covalente avec ladite première séquence, le conjugué peptide-polynucléotide de la première séquence peut alors répondre à la formule suivante :

$$AA_1 \text{ ------ } AA_m\text{-}N_1 \text{ ------ } N_n \qquad (I)$$

dans laquelle
n est compris entre 200 et 1, mais le plus souvent entre 4 et 50, de préférence 10

$m = 2n + p$ et

p est un nombre entier supérieur ou égal à 1, par exemple égal à environ 20 et
dans laquelle
AAi représente les acides aminés et
N les nucléotides.

Lors de l'hybridation avec une séquence complémentaire $N'_1$---$N'_{p+n}$ le complexe d'hybridation se présente selon la configuration suivante :

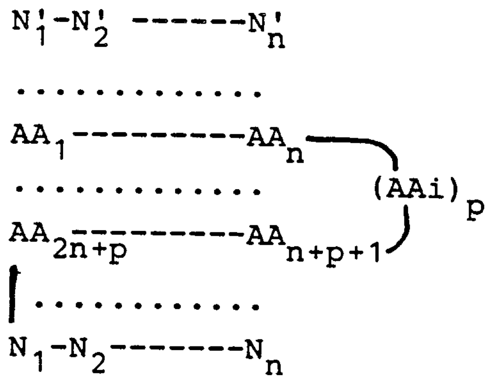


(1)

Les lignes pointillées "...." schématisent l'interaction entre acides aminés et bases et l'interaction entre les deux chaînes peptidiques.

$(AAi)_p$ représente la séquence "charnière", par exemple p acides aminés identiques ou différents.

La configuration (1) schématisée ci-dessus peut également se produire sans que le peptide soit lié à la séquence oligonucléotidique.

Dans la présente demande,

. la séquence $N_1$-$N_2$ ----- $N_n$ correspond à la convention de sens 5' → 3', et $N_1'$ -$N_2'$ ----$N_n$ au sens 3' → 5',

. les enchaînements d'acides aminés $AA_1$-$AA_2$----- $AA_m$ (1→m) correspondent à l'extrémité $NH_2$ libre pour $AA_1$ et COOH libre pour $AA_m$, c'est-à-dire qu'un acide aminé $AA_x$ correspond à ($NH_2$-)AAx(-COOH).

De préférence, le cas échéant, la liaison entre le peptide et l'oligonucléotide se fait au départ entre un nucléotide portant une thymine ou uracile triazolée de l'oligonucléotide que l'on aura ajouté si nécessaire à l'extrémité 5' de l'oligonucléotide et une lysine du peptide que l'on aura ajoutée si nécessaire à l'extrémité C terminale du peptide, selon le procédé suivant:

1) le groupe oxo de la thymine ou de l'uracile est substitué sur son carbone en position 4 par un groupe triazole,

2) le groupe triazole est substitué par ladite lysine et partant par le peptide, par l'intermédiaire du groupe $NH_2$ à l'extrémité $\epsilon$ de la chaîne de ladite lysine.

Finalement, après fixation, la thymine ou l'uracile triazolées deviennent une méthyl-cytosine ou une cytosine fonctionnalisée respectivement et l'oligo-nucléotide est lié au peptide par une méthyl-cytosine ou une cytosine respectivement.

On peut schématiser l'épingle à cheveux (C) entre ladite première séquence ou séquence sonde (A) et ladite seconde séquence ou séquence cible (B) de la manière suivante

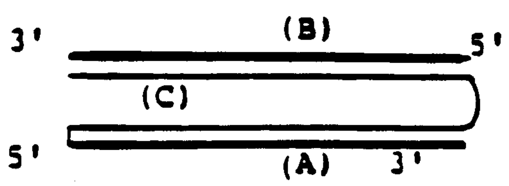


(2)

Lesdites première et seconde séquences peuvent constituer une partie seulement des séquences sonde et cible respectivement, c'est-à-dire que le complexe d'hybridation peut se schématiser ainsi

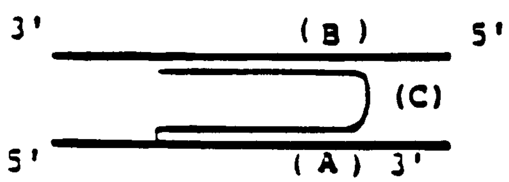


(3)

Dans ce dernier cas de figure, un conjugué peptide-polynucléotide de la première séquence peut être représenté comme suit :

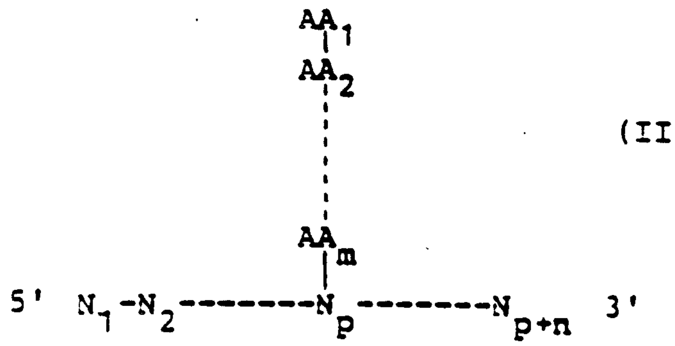

(II)

formule dans laquelle

- p est compris entre 100 et 1, le plus souvent entre 5 et 25
- n est compris entre 100 et 0, le plus souvent entre O et 20
- m est compris entre 400 et 3, le plus souvent entre 10 et 110, de préférence environ 20

(lorsque p = 1, on retrouve la formule (I)).

Selon une autre variante de réalisation, le peptide comporte :

- une première succession d'acides aminés qui présentent une interaction un à un respectivement avec une succession de bases de nucléotides de ladite première séquence,
- une seconde succession d'acides aminés qui présentent une interaction un à un respectivement avec une succession de bases de nucléotides de ladite seconde séquence,
- une troisième succession d'acides aminés qui présentent une interaction un à un respectivement avec une succession de bases de nucléotides de ladite première séquence nucléotidique,
- lesdites première et seconde successions d'acides aminés d'une part, et lesdites seconde et troisième successions d'acides aminés d'autre part, étant reliées à chaque fois par une séquence dite "charnière" constituée notamment par un ou plusieurs acides aminés n'interagissant pas avec les bases desdites première et seconde séquences nucléotidiques,
- les séquences de nucléotides dont les bases interagissent avec ladite seconde succession d'acides aminés constituant la séquence complémentaire totale ou partielle de celles dont les bases interagissent avec lesdites première et troisième successions d'acides aminés,

  de telle sorte que,
- les acides aminés desdites première et troisième successions d'aminoacides interagissent de manière coopérative avec les acides aminés de ladite seconde succession,
- l'interaction coopérative résultante entre le peptide et l'hélice double brin formée par le complexe d'hybridation desdites première et seconde séquences nucléotidiques étant telle que le peptide se glisse dans le grand sillon de la double hélice et prenne une structure en double épingle à cheveux entre ladite première et ladite seconde séquences nucléotidiques par l'intermédiaire des deux séquences "charnières".

Selon cette variante de réalisation de l'invention, on peut schématiser le complexe d'hybridation de la manière suivante :

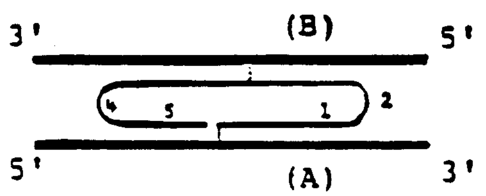


(4)

1 représente la première succession d'aminoacides

3 représente la seconde succession d'aminoacides

2 représente la première série d'acides aminés "charnière" entre 1 et 3

5 représente la troisième succession d'aminoacides dans ce mode de réalisation de l'inven-

tion, et

[4] représente les acides aminés "charnière" entre [3] et [5]

(A) et (B) représentent respectivement lesdites première et seconde séquences.

La configuration (4) peut encore se produire sans que nécessairement le peptide soit lié à ladite première séquence.

On peut, selon un autre aspect de l'invention, fixer le peptide sur ladite première séquence par l'intermédiaire de deux liaisons, c'est-à-dire par chacune des extrémités du polypeptide sur le polynucléotide de ladite première séquence, comme décrit précédemment.

Le complexe pourra alors être schématisé comme suit :

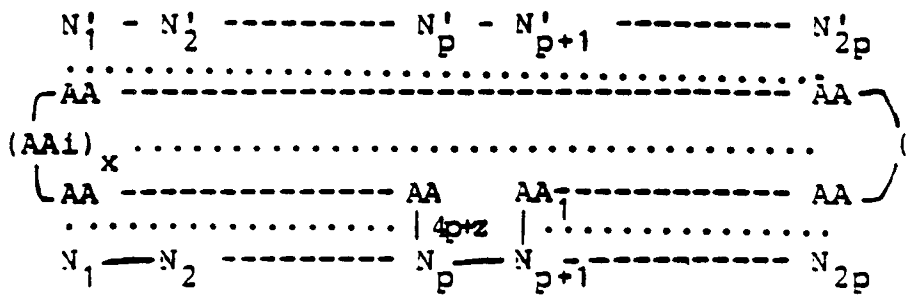

(5)

dans laquelle z = x + y

$(AAi)_x$ représente une séquence dite "charnière", par exemple x acides aminés "charnières"

$(AAj)_y$ représente une séquence dite "charnière" par exemple y acides aminés "charnières"

D'une manière générale, les nucléotides de ladite première séquence peuvent comporter des bases choisies parmi l'adénine, la guanine, la thymine, la cytosine, l'uracyle, et toutes autres bases puriques telles que l'inosine, la 2-6 diaminopurine ou la 6 amino 8-bromopurine, ou pyrimidiques, telle que le 5-bromo uracyle ou le 5-iodo uracyle.

Avantageusement donc la séquence oligonucléotidique sonde comportera une cytosine terminale.

Par ailleurs, du fait de la ressemblance entre les motifs exprimés par l'uracyle et la cytosine il est préférable d'éviter les interactions entre un acide aminé et l'uracyle, on choisira donc des séquences cibles de préférence avec le moins possible d'uracyle et par conséquent, une séquence sonde avec le moins possible d'adénine.

Les peptides se complexent sur des hélices nucléotidiques de préférence de forme A, mais des interactions avec des hélices B peuvent aussi être envisagées.

Les différents acides aminés constituant le peptide sont choisis en fonction de la nature des bases qui se succèdent sur les nucléotides de séquences d'acides nucléiques concernés comme mentionné cidessus.

Lorsque la base est une thymine, l'acide aminé doit être très hydrophobe, de préférence. L'acide aminé peut donc être choisi parmi entre autres la L-phénylalanine, la L-leucine, la L-isoleucine, la L-valine, la L-alanine, la L-méthionine. De préférence encore, on la choisira parmi la L-leucine, la L-isoleucine et la L-valine.

On évitera les acides aminés hydrophiles, tels que la L-sérine, la L-thréonine, la L-histidine, la L-asparagine, la L-glutamine, la L-lysine ou la L-arginine.

Lorsque la base est un uracyle, l'acide aminé doit être un acide aminé dit "ambigu" ou "amphiphile", c'est-à-dire ne présentant pas d'effets solvants, soit ni trop hydrophobes ni trop hydrophiles, ou un acide aminé acide.

On peut citer, lorsque la base est un uracyle, les acides aminés suivants : L-alanine, l'acide L-aspartique, l'acide L-glutamique, L-sérine, L-cystéine, L-thréonine, mais de préférence on citera la L-alanine, la L-sérine et la L-thréonine.

On évitera la L-isoleucine, la L-leucine, la L-asparagine, la L-glutamine, la L-lysine et la L-arginine.

Lorsque la base est une cytosine, on choisira des acides aminés "ambigus" ou "amphiphiles", c'est-à-dire ne présentant pas d'effet solvant soit ni trop hydrophobes, ni trop hydrophiles.

On peut citer notamment la L-alanine, la L-sérine, la L-cystéine, la L-thréonine, la L-tyrosine, la L-histidine, mais de préférence on utilisera la L-alanine, la L-sérine, la L-thréonine.

Lorsque la base est une adénine ou une guanine, on utilisera des acides aminés hydrophiles. On peut citer notamment la L-tyrosine, la L-histidine, la L-asparagine, la L-glutamine, la L-lysine, la L-arginine, la L-acide aspartique, la L-acide glutamique, la L-sérine, la L-cystéine, la glycine, la L-thréonine.

Plus précisément, lorsque la base est une adénine, on choisira de préférence des acides aminés très chargés et très hydrophiles, notamment la L-asparagine, la L-glutamine ou la L-lysine.

Lorsque la base est une guanine, on choisira des acides aminés petits et hydrophiles, de préférence la L-sérine et la L-cystéine, ainsi que la L-arginine et la L-lysine.

Lorsque la base est une adénine, on évitera donc les acides aminés hydrophobes, tels que la L-leucine, la L-isoleucine, la L-valine, la L-phénylalanine et la L-alanine.

Lorsque la base est une guanine, on évitera les acides aminés hydrophobes, tels que la L-phénylalanine, la L-leucine, la L-isoleucine, la L-valine, la L-alanine.

Si la base est une autre pyrimidine, l'acide aminé doit être choisi entre autres et de préférence parmi les acides aminés conseillés pour la thymine, l'uracyle ou la cytosine.

Si la base est une autre purine, l'acide aminé doit être choisi entre autres et de préférence parmi les acides aminés conseillés pour l'adénine ou la guanine.

D'autres acides aminés non naturels peuvent être utilisés soit pour augmenter l'affinité du peptide pour l'hélice, soit pour apporter des propriétés nouvelles au peptide.

Certains enchaînements d'acides aminés ne permettent pas une interaction stable avec une séquence précise d'ADN ou ARN double brin, notamment on évitera les enchaînements suivants dans la mesure du possible :

| | | |
|---|---|---|
| $AA_x$-Cys-Asp-$AA_z$ | $AA_x$-Cys-Asn-$AA_z$ | $AA_x$-Cys-Glu-AAz |
| AAx-Cys-Gln-AAz | AAx-Asp-Cys-AAz | AAx-Asn-Cys-AAz |
| AAx-Glu-Cys-AAz | AAx-Gln-Cys-AAz | |
| AAx-Asp-Trp-AAz | AAx-Glu-Trp-AAz | |
| AAx-Trp-Asp-AAz | AAx-Trp-Glu-AAz | |
| AAx-Asp-Gly-AAz | AAx-Asn-Gly-AAz | |
| AAx-Gly-Asp-AAz | AAx-Gly-Asn-AAz | |
| AAx-Glu-Gly-AAz | AAx-Gln-Gly-AAz | |
| AAx-Gly-Glu-AAz | AAx-Gly-Gln-AAz | |
| AAx-Ser-Asp-AAz | AAx-Ser-Glu-AAz | |
| AAx-Thr-Asp-AAz | AAx-Thr-Glu-AAz | |
| AAx-Glu-Thr-AAz | AAx-Asp-Ser-AAz | |
| AAx-Glu-Ser-AAz | AAx-Asp-Thr-AAz | |

AAx et AAz représentent des acides aminés en amont et en aval respectant la convention

$(NH_2)$-AAx ------ AAz(-COOH)

La charnière de l'épingle à cheveux est constituée de préférence de 2, 3 ou 4 acides aminés. Ils seront de préférence choisis parmi la glycine, la L-asparagine et la L-glutamine.

Dans son aspect le plus général, la présente invention a pour objet un conjugué constitué par une séquence polynucléotidique d'ADN ou d'ARN modifiés ou non, à configuration $\alpha$ ou $\beta$ , et un peptide dont les acides aminés sont choisis de telle sorte que le peptide prenne une structure en épingle à cheveux ou en double épingle à cheveux dans le grand sillon de la double hélice d'hybridation que forme ladite séquence polynucléotidique avec une séquence d'ADN ou d'ARN complémentaire.

En particulier, les acides aminés constituant ledit peptide sont choisis en fonction de la nature des nucléotides constituant ladite séquence polynucléotidique compte tenu des principes de correspondance énumérés précédemment, ladite séquence polynucléotidique correspondant à ladite première séquence d'ADN ou d'ARN, le peptide étant lié ou non de façon covalente audit polynucléotide.

La présente invention a également pour objet des conjugués peptide-polynucléotide utiles dans le procédé selon l'invention, conjugués de formule générale (I) :

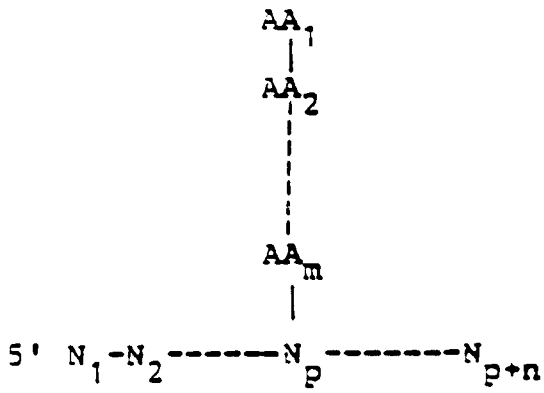

(I)

formule dans laquelle en général :

$100 > p \geqq 1$, le plus souvent $25 > p \geqq 5$

$100 > n \geqq 0$, le plus souvent $25 > n \geqq 0$

$400 > m \geqq 3$, le plus souvent $100 > m \geqq 10$

$-AA_1$-----$AA_m$ représente un peptide,

$-N_1$------$N_{p+n}$ représente un polynucléotide.

Les acides aminés AA étant choisis en fonction de la nature des nucléotides N constituant le polynucléotide compte tenu des principes de correspondance énumérés précédemment, de manière à ce que le peptide adopte une structure en épingle à cheveux entre ladite première séquence et ladite seconde séquence dans le grand sillon de la double hélice polynucléotidique par l'intermédiaire d'une séquence dite "charnière" constituée notamment par un ou plusieurs acides aminés n'interagissant pas avec les bases des nucléotides.

Comme il ressort de la formule I ci-dessus, le peptide est fixé sur le polynucléotide non nécessairement à une des extrémités de celui-ci. Toutefois, de préférence, cette fixation aura lieu à une des extrémités du polynucléotide, on aura alors $p = 1$ dans la formule I ci-dessus.

Les acides aminés doivent avantageusement respecter les règles de principe de concordances énumérées précédemment vis-à-vis des nucléotides desdites première et seconde séquences d'une part, et vis-à-vis des acides aminés voisins dans la chaîne peptidique d'autre part.

Toutefois, il n'est pas obligatoire que tous les acides aminés respectent ces règles de principe dans la mesure où une proportion suffisante pour assurer la stabilité de l'hybridation y répond. On peut notamment citer une proportion de 30 %.

On notera en outre que le peptide n'est pas nécessairement fixé au polynucléotide par l'intermédiaire d'un acide aminé terminal, contrairement à la réalisation préférentielle qui est représentée sur la formule I.

De même, la ou les charnière(s) n'est pas ou ne sont pas nécessairement constituée(s) d'une chaîne d'acides aminés, mais peuvent être des groupements chimiques non peptidiques.

La présente invention a également pour objet un conjugué peptide-polynucléotide utile dans un procédé selon l'invention, caractérisé en ce qu'il répond à la formule générale II

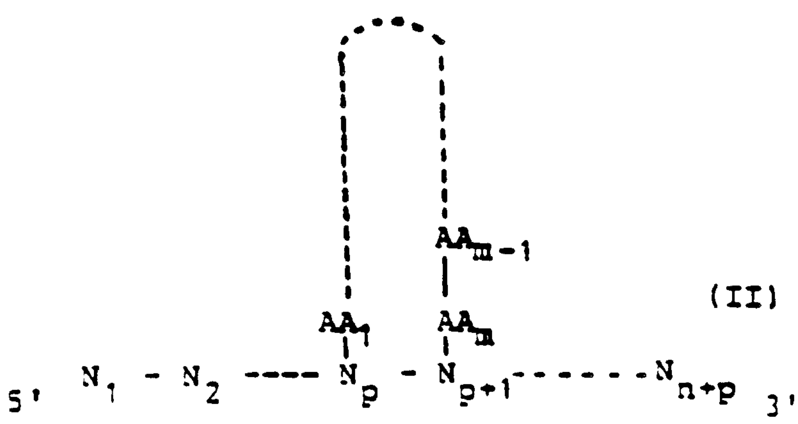

(II)

dans laquelle

- $AA_1$ ---------- $AA_m$ représente m acides aminés du peptide
- $N_1$ ---------- $N_{p+n}$ représente ladite première séquence de p + n nucléotide
- $100 > p \geqq 1$, le plus souvent $25 > p \geqq 5$
- $100 > n \geqq 1$, le plus souvent $25 > n \geqq O$
- $400 > m \geqq 3$, le plus souvent $110 > m \geqq 10$, de préférence environ 20.

les acides aminés de $AA_1$ ---- $AA_m$ étant choisis conformément aux règles de correspondance mentionnées précédemment, de manière à ce que le peptide adopte une structure en double épingle à cheveux grâce à deux séquences "charnières" en son sein.

Dans ce conjugué $AA_m$ peut être lié à $N_{p+1}$ par un lien amide entre le COOH de $AA_m$ et un $NH_2$ de la base. En outre, $AA_1$ peut être lié à $N_P$ par un lien amide entre un COOH de $AA_1$ et un $NH_2$ de la base si $AA_1$ représente un acide glutamique ou un acide aspartique. On peut sinon relier un $NH_2$ de $AA_1$ et celui de la base par un "linker" chimique consistant par exemple en un groupement chimique présentant deux fonctions COOH, telles qu'un groupement hémisuccinyl, chacune des fonctions COOH du "linker" formant un lien amide avec les fonctions $NH_2$ de l'acide aminé et de la base respectivement.

Toutefois, de préférence, les liens entre le peptide et le polynucléotide se feront comme décrit précédemment entre une lysine et une thymine ou uracile triazolée. Dans ce cas, $AA_m$ et $AA_1$ représentent donc des lysines et $N_{p+1}$ et $N_p$ représentent des méthyl-cytosine, ou cytosine respectivement.

Dans un mode de réalisation approprié des conjugués selon l'invention, ceux-ci peuvent être représentés par une formule générale III

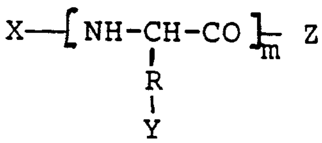

(III)

dans laquelle

- m est un nombre entier compris entre 3 et 400.
- R est choisi parmi les chaînes'd'acides aminés naturels éventuellement modifiés et les chaînes d'acides aminés non naturels éventuellement modifiés.
- Z est choisi parmi le radical OH, un polynucléotide, une molécule de protection et une molécule de marquage telle que notamment le dinitrophénol, la biotine, la fluorescéine entre autres ....
- X est choisi parmi le radical H, un polynucléotide, une molécule de protection et une molécule de marquage telle notamment le dinitrophénol, la biotine, la fluorescéine, entre autres.
- Y est présent lorsque R est la chaîne de la lysine, de l'acide aspartique ou glutamique auquel cas Y peut représenter un polynucléotide, une molécule de protection, ou une molécule de marquage, notamment le dinitrophénol, la biotine, la fluorescéine, ces éléments étant fixés sur la fonction $NH_2$ ou COOH de R.

L'un seulement de X, Y et Z est un polynucléotide correspondant à ladite première séquence.

Cette alternative entre X, Y et Z pour représenter le polynucléotide témoigne de la triple possibilité de fixer celui-ci sur le peptide, soit par l'intermédiaire d'une fonction COOH du peptide sur le $NH_2$ d'une base, soit d'une fonction $NH_2$ du peptide sur une base de l'oligonucléotide, en particulier d'une fonction $NH_2$ située en bout de chaîne d'une lysine du peptide sur une thymine ou uracile triazolée de l'oligonucléotide tel que mentionné précédemment.

Le peptide peut encore être lié à un oligonucléotide par l'un des sites réactifs dudit oligonucléotide ou l'un de ses sites modifiés, selon l'une des autres méthodes suivantes.

La synthèse des complexes peptide-oligonucléotide est connue dans l'art antérieur, on pourra faire appel aux techniques décrites d'une part par Barbara Chu (Nucleic Acid Research, 1983, 11, 6513-6529), d'autre part par Dannagupta et al. (EP 154 884) reprises par Lemaître et coll. (FR 2 582 653).

On pourra notamment effectuer des liaisons sur les sites réactifs, tels que :

. le radical 2'OH d'un ribose s'il s'agit d'un ARN
. le radical 3'OH d'un ribose s'il s'agit d'un ARN ou d'un ADN
. le radical 5'OH d'un ribose
. le radical OH du phosphate en 3'
. le radical OH du phosphate en 5'
. le radical $NH_2$ en C6 de l'adénine
. le radical $NH_2$ en C2 de la guanine
. le radical $NH_2$ en C4 de la cytosine.

ces radicaux $NH_2$ seront fixés par exemple sur la fonction carboxyle du peptide par l'intermédiaire d'un lien amide.

La méthode selon Barbara Chu consiste à conjuguer une fonction $NH_2$ d'un acide aminé terminal du peptide avec le radical OH du phosphate en 5'.

La méthode de Lemaître et coll. consiste à modifier le cycle ribose d'ARN pour introduire deux fonctions aldéhydes sur les carbones en 2' et 3', l'alcoylation réductive du composé obtenu par un polypeptide sur sa fonction $NH_2$ permettant le couplage par l'intermédiaire d'une cyclisation sous forme d'un hétérocycle avec un hétéroatome d'azote.

La synthèse des peptides est connue et fait appel aux techniques classiques utilisant soit les voies du génie génétique, soit les voies du génie chimique sur support solide selon Merrifield ou en phase liquide.

La synthèse des oligonucléotides est également connue et fait appel aux techniques classiques utilisant soit les voies du génie chimique, soit celles du génie génétique.

La présente invention a donc également pour objet des conjugués caractérisés en ce que la liaison entre le peptide et le polynucléotide se fait entre une lysine du peptide et une méthyl-cytosine ou cytosine du polynucléotide, le $NH_2$ situé à l'extrémité de la chaîne de la lysine étant fixé sur le carbone en position 4 de la méthyl-cytosine ou cytosine respectivement.

De même, la présente invention a pour objet un procédé de préparation des conjugués selon l'invention, caractérisé en ce que le peptide est lié au polynucléotide en substituant le groupe triazole d'une thymine ou uracile triazolée en position 4 du polynucléotide de départ, par le peptide via son groupe $NH_2$ situé à l'extrémité de la chaîne d'une lysine.

Enfin, la présente invention a pour objet l'application du procédé et des conjugués selon l'invention.

Il peut s'agir d'une application thérapeutique ou biotechnologique dans le cas d'une utilisation du procédé ou des conjugués au blocage ou au déblocage d'un gène de structure ou de régulation situé sur l'ADN d'un organisme quelconque.

Il peut également s'agir d'une application thérapeutique ou biotechnologique dans le cas d'une utilisation du procédé ou de ces conjugués à l'inhibition de la traduction d'un ARN messager ou non ainsi qu'à l'inhibition des processus d'épissage des ARN messagers.

Les applications du procédé ou de ces conjugués peuvent également être des applications comme sondes diagnostiques, dans ce cas, l'un de X ou Z doit représenter une molécule de marquage.

Une application biotechnologique peut également consister en l'utilisation du procédé ou de ces conjugués, comme outil de purification de matériel nucléique.

On peut compléter le matériel génétique d'une cellule sous quelle que forme que ce soit de telle façon que cette cellule produise par exemple une molécule antisens et le peptide qui la stabilise sur sa cible, sans pour autant qu'une liaison covalente existe entre le peptide et l'oligo-nucléotide, notamment pour une application dans le domaine des plantes transformées.

En revanche, pour une application en thérapeutique humaine il sera nécessaire que le peptide soit lié à l'oligo-nucléotide.

D'autres caractéristiques et avantages de la présente invention apparaîtront à la lumière des exemples qui vont suivre qui font référence à des figures.

Sur la figure 1, sont représentés deux groupes :NH de deux acides aminés qui coopèrent entre eux et interagissent dans le grand sillon avec une paire de bases T - A hybridée.

Sur la figure 2, sont représentés deux groupes :NH de deux acides aminés qui coopèrent entre eux et interagissent dans le grand sillon avec une paire de bases C - G hybridée.

Sur la figure 3, est représentée l'interaction du complexe d'hybridation incorporant le peptide selon l'invention.

EXEMPLE 1 : INTERACTION ET COOPERATION

Sur les figures 1 et 2, (1) représente le site de fixation des bases aux cycles riboses.

Sur les figures 1 et 2, on voit que chaque azote d'une liaison peptidique (2a et 2b) de chacun des deux acides aminés et à l'origine de deux liaisons hydrogènes, une comme accepteur d'hydrogène, appelé "E" par le biais de l'électron, et une comme donneur d'hydrogène, appelé "H". L'azote d'un groupe NH d'un acide aminé interagit avec l'hydrogène d'un groupe NH de l'autre acide aminé. De même, l'électron de l'azote de cette autre acide aminé interagit avec le $NH_2$ en $C_6$ de l'adénine (figure 1) ou le $NH_2$ en $C_4$ de la cytosine (figure 2). L'hydrogène de l'azote du premier acide aminé interagit avec l'$O_4$ de la thymine (figure 1) ou l'$O_6$ de la guanine (figure 2).

Le radical R de l'acide aminé de série L représenté figure 3 interagit avec le reste de la surface exposée au grand sillon de la base du côté amine de l'acide aminé. Les flèches symbolisent cette

interaction.

Sur la figure 3, $B_1$ et $B_2$ représentent deux bases de nucléotides de ladite première séquence et $B'_1$ et $B'_2$ représentent les deux bases complémentaires de $B_1$ et $B_2$, bases de nucléotides de ladite seconde séquence.

$B_1$ peut représenter par exemple une thymine, $B'_1$ représentant alors l'adénine. Ces deux bases interagissent notamment par l'intermédiaire de l'$O_4$ de la thymine et du $NH_2$ en $C_6$ de l'adénine comme représenté figure 1.

$B_2$ peut représenter par exemple une guanine, $B'_2$ représentant alors la cytosine; ces deux bases interagissant notamment par l'intermédiaire de l'$O_6$ de la guanine et du $NH_2$ en $C_4$ de la cytosine comme représenté figure 2.

EXEMPLE 2 : CONJUGUE PEPTIDE-OLIGONUCLEOTIDE

On donne ci-après un exemple de réalisation de l'invention pour un agent antiparasitaire, notamment contre la trypanosomiase, la molécule étant dirigée contre une partie d'une séquence consensus de 35 nucléotides ajoutée à l'extrémité 5' de tous les ARNm de ce parasite :

5' AAC GCT ATT ATT AGA ACA GTT TCT GTA CTA TAT TG 3'.

La séquence mentionnée ci-dessus correspond à la séquence au niveau de l'ADN.

Un exemple de conjugué peptide-oligonucléotide et de complexe d'hybridation est donné ci-dessous; la séquence cible correspondant à la traduction ARN de la séquence donnée précédemment :

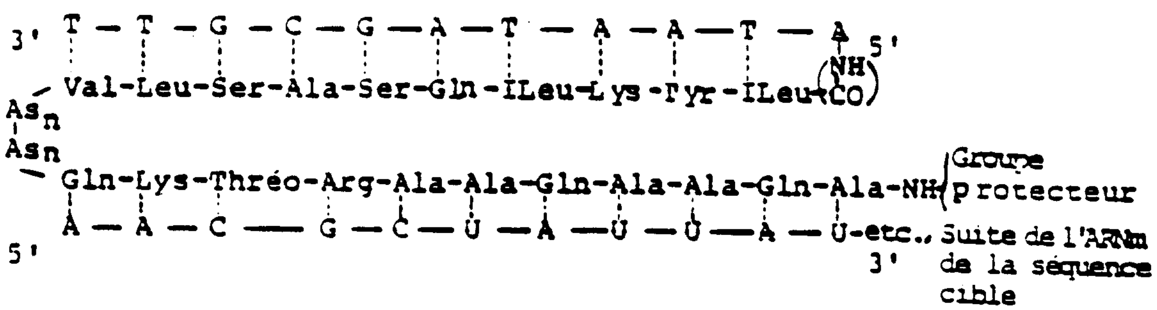


Outre l'application antiparasitaire pour la trypanosomiase mentionnée ci-dessus, on peut également utiliser des conjugués selon l'invention dans une action antiparasitaire contre la leishmaniose ou une action antivirale dans le cas de $HiV_1$, $HiV_2$, d'Herpès, etc., ou encore antibactérienne pour la lèpre, la tuberculose, etc.

Une application à l'inhibition de la traduction des ARNm peut être envisagée dans le cas :

- du récepteur $H_1$ (ulcère)
- de la rénine (hypertension artérielle)
- de l'angiotensine (hypertension artérielle).

Ce type d'application où la séquence cible est de l'ARN est particulièrement intéressant car la double hélice d'hybridation ADN-ARN adopte une forme A sur laquelle les peptides se complexent plus facilement compte tenu du grand sillon de ces hélices A.

EXEMPLE 3 : SYNTHESE D'UN OLIGONUCLEOTIDE COUPLE A UN PEPTIDE

Préparation des solvants

La pyridine est portée 2 heures au reflux sur hydrure de calcium, distillée, portée au reflux avec le chlorure de l'acide paratolulène sulfonique puis redistillée.

1) Préparation de thymine triazolée pour la synthèse manuelle (phosphotriester)
(selon la méthode décrite par W.L. Sung : Nucleic Acid Research (1981), vol 9, p 6139 et J. Org. Chem. (1982), vol 47, p 3623).

On prend 4 g d'O-di-méthoxytrityl-5'O-[(2-chlorophényl) ($\beta$-cyanoéthyl)phosphate]-3' désoxy-2' thymidine (=DMTr Tpce) (PM = 787,5; 5,07 mmoles) préparé selon la méthode décrite par M.J. Gait ("Oligonucleotide synthesis : a practical approach", 1985, IRL Press) et 2,5 g o-chlorophényl dichlorophosphate (PM = 245,33 ; 10,1 mmoles) et 1,4 g 1,2,4 triazole (PM = 69,07 ; 20,3 mmoles).

On agite la solution dans 9 ml de pyridine déshydratée à 10°C pendant 5 minutes puis à la température de la pièce pendant un week-end.

On dilue le mélange avec 45 ml d'$H_2O$ et extrait deux fois dans 75 ml $CH_2Cl_2$ désacidifié.

On lave des phases organiques combinées dans 2 % $NaHCO_3$ aqueux, puis séchage avec $Na_2SO_4$, filtrage et évaporation sous vide.

On resuspend le précipité dans un ballon avec 2 ml de $CH_2Cl_2$, puis on verse rapidement 100 ml d'éther de pétrole, on laisse reposer, on enlève l'éther usagé et on remet 100 ml d'éther propre afin d'enlever toute trace de pyridine qui aurait l'inconvénient d'empêcher la séparation des produits.

Le produit est chromatographié sur une colonne de silice Merck 9385 ou 7734 tassée dans le $CH_2Cl_2$.

On élue d'abord avec le $CH_2Cl_2$ 100 % puis avec $CH_2Cl_2$ + 1 % méthanol, puis $CH_2Cl_2$ + 2 % méthanol, puis $CH_2Cl_2$ + 3 % méthanol puis $CH_2Cl_2$ + 4 % méthanol et enfin avec $CH_2Cl_2$ + 5 % méthanol.

Les contrôles de sortie de colonnes se font par chromatographie sur couches minces (CCM) effectuées sur des plaques de silice Merck $60F_{254}$ (0,2 mm). L'avantage du triazole est qu'il fluoresce à 366 nm ce qui permet de le suivre très facilement. Les sorties de colonne, fluorescentes à 366 nm,

sont repassés afin d'obtenir un produit parfaitement pur. A la fin de la quatrième colonne, lorsque l'on arrive au 5 % méthanol, on obtient une seule tache.

2) Couplage en synthèse manuelle du nucléoside modifié à un oligonucléotide

Couplage d'un oligo (n-1) à la machine Applied Biosystems dans un premier temps.

On sort la cartouche de la machine et la décortiquer.

Mettre les billes sur un petit fritté.

Décyanoéthyler le DMTr Tpce avec un mélange de Triéthylamine, Pyridine et Eau (TPE 1/3/1) pendant 20 minutes.

On couple ensuite la thymine triazolée 3'OH, comme un nucléotide normal à l'oligo 5'OH avec 500 µmoles de (triisopropyl-2,4,6 benzène sulfonyl)-1 nitro-3 triazole-1,2,4 (TPNST) dans la pyridine anhydre pendant 2 heures.

Les fonctions 5' restées libres sont bloquées avec une solution d'anhydride acétique dans la pyridine (10 % v/v) pendant 30 minutes à température ambiante.

On rince plusieurs fois à la pyridine puis au $CH_2Cl_2$-$CH_3OH$ (90:10 v/v).

Détrityler l'extrémité 5' avec de l'acide benzène sulfonique à 2 % dans un mélange $CH_2Cl_2$.

On répète l'opération jusqu'à complète détritylation.

On lave l'oligonucléotide plusieurs fois avec de la pyridine.

3) Préparation de thymine triazolée pour la synthèse automatique (phosphoramidite)
(selon la méthode décrite par T.R. Webb et M.D. Matteucci, Nucleic Acids Res., 1986, vol 14, p 7661)

Mettre 1,4 g de 1,2,4-triazole (PM = 69,07 ; 20,3 mmoles) dans 30 ml d'acétonitrile.

On refroidit le mélange dans un bain de glace.

On ajoute très lentement et sous bonne agitation 500 µl de chlorure de phosphoryle distillé ($POCl_3$) (5,4 mmoles) qui font apparaître un précipité blanc.

On ajoute ensuite, goutte à goutte, 3 ml de triéthylamine (21,7 mmoles).

On agite le mélange réactionnel dans un bain de glace pendant 30 minutes.

On ajoute 1 g d'O-di-méthoxytrityl-5' O-[(2-chlorophényl) (β-cyanoéthyl) N,N'-(diisopropyl) phosphoramidite]-3' désoxy-2' thymidine (PM = 793,65 ; 1,26 mmole) fourni par la Société Applied Biosystems, dissoute dans 5 ml d'acétonitrile.

On agite 30 minutes à température ambiante.

On laisse incuber pendant 48 heures.

La réaction est arrêtée avec une solution à 5 % de bicarbonate de sodium.

La phase aqueuse est extraite plusieurs fois avec du dichlorométhane, séchée sur sulfate de sodium, filtrée puis évaporée à sec.

La (1,2,4-triazol-1-yl)-4 O-diméthoxytrityl-5' O-[(β-cyanoéthyl) N,N'-(diisopropyl) phosphoramidite]-3' désoxy-2' thymidine est dissoute dans une très petite quantité de dichlorométhane puis reprécipitée dans l'éther de pétrole à 70°C.

4) Couplage en synthèse automatique du nucléoside modifié à l'oligonucléotide

Le DMTr Tpce est couplé à l'extrémité 5' de l'oligonucléotide fixé sur la résine comme un nucléoside normal.

5) Préparation du peptide

Les différents peptides sont synthétisés avec un synthétiseur Applied Biosystems en phase solide selon les méthodes classiques. Ceux-ci sont purifiés sur HPLC avec un gradient de tampon A et de tampon B (tampon A : acétate d'ammonium 100 µM, tampon B : 20 % acétate d'ammonium 100 µM et 80 % acétonitrile). Les colonnes utilisées pour analyser les produits contiennent un support de phase inverse de type HS3 C18 ou Nucléosil µC18. Les peptides destinés à être liés à un oligonucléotide sont fournis sans protecteur et ont leurs extrémités N-terminale acétylée et C-terminale amidée.

6) Préparation du conjugué oligonucléotide-peptide

Le peptide est accroché par le $NH_2$ situé à l'extrémité de la chaîne de la lysine C-terminale au nucléotide par une réaction de substitution du triazole.

On met dans 500 µl de pyridine l'oligonucléotide fixé sur les billes et le peptide dans les proportions 1:3 à 1:10, à température ambiante pendant 48 à 72 heures.

On lave les billes 4 à 5 fois avec de la pyridine puis on les sèche.

On détache le conjugué oligonucléotide-peptide des billes ainsi que les éléments protecteurs des nucléotides avec une solution d'ammoniaque à 25 % pendant 5 heures à 56 °C.

On prélève le surnageant après centrifugation puis on le sèche.

Le produit resuspendu dans de l'eau est purifié sur HPLC par un gradient de tampon A et tampon B.

Le pic majoritaire (sorti à 13 % pour le peptide n° 1) est caractérisé par l'hydrolyse acide d'un petit aliquot. L'analyse d'acides aminés permet de s'assurer que le peptide est bien fixé à l'oligonucléotide.

Un spectre UV permet de visualiser la présence de l'oligonucléotide à 250 nm. Le rendement de couplage se situe entre 80 et 85 %. Le rendement de purification est seulement de 20 %.

N.B. : après fixation du peptide la thymine triazolée devient une méthyl-cytosine fonctionnalisée.

EXEMPLE 4 : Mesure de l'effet stabilisateur de différentes séquences peptidiques sur différentes séquences oligonucléotidiques

L'effet de stabilisation d'un peptide sur une hélice oligo-nucléotidique A est mis en évidence par l'élévation de la température de demi-transition des complexes. La mesure se fait dans une solution à 66 % éthanol et 6,5 mmol de phosphate de sodium à pH 7,4.

Les séquences suivantes ont été étudiées :

1) Oligonucléotide-peptide 1 :

Acétyl-NH-Asn-Thréo-Arg-Ser-Arg-Ala-Ala-Ala-Arg-Ala-Ser-Ser-Gly-Gly-Ala-Ala-Arg-Ala-Ser-Gly-Arg-Ser-Phe-Val-Ser-Lys-$CONH_2$

I

3'CCGCGGGCTTGT Cme 5'

Oligonucléotide complémentaire : 5'GGCGCCCGAACAG 3'

Oligonucléotide témoin 1 bis : 3'CCGCGGGCTTGTC 5'

Oligonucléotide complémentaire1: 5'GGCGCCCGAACAG 3'

2) Acétyl-NH-Asn-Ala-Ala-Ala-Ala-Ser-Lys-Gln-Thréo-Arg-Ser-Ser-Gly-Gly-Ser-Phe-Val-Ser-Phe-Leu-Ala-Ser-Ser-Ser-Ser-Lys-$CONH_2$

I

3'CCTTGTTCGGGGT Cme 5'

Oligonucléotide complémentaire 2 : 5'GGAACAAGCCCCAG 3'

Oligonucléotide témoin 2 bis : 3'CCTTGTTCGGGGTC 5'

Oligonucléotide complémentaire 2 : 5'GGAACAAGCCCC AG 3'

3) Peptide autocomplémentaire 3 non fixé sur l'oligonucléotide 3

H-Arg-Gln-Ala-Ser-Leu-Ala-Arg-Gln-Ala-Ser-Leu-Ala-OH

OH-Ala-Leu-Ser-Ala-Gln-Arg-Ala-Leu-Ser-Ala-Gln-Arg-H

Oligonucléotide complémentaire 3 : 3'GACGTCGACGTC 5'

5'CTGCAGCTGCAG 3'

4) Peptide autocomplémentaire 4 non fixé sur l'oligonucléotide 4

H-Ala-Leu-Ala-Ser-Lys-Ser-Ala-Leu-Ala-Ser-Lys-Ser-OH

OH-Ser-Lys-Ser-Ala-Leu-Ala-Ser-Lys-Ser-Ala-Leu-Ala-H

Oligonucléotide autocomplémentaire : 3'CTCGAGCTCGAG 5'

5'GAGCTCGAGCTC 3'

Ainsi, pour cette dernière expérience sans peptide la température de demi-transition n'est que de 32 °C alors qu'avec peptide la température de demi-transition s'élève à 48 °C témoignant d'une plus forte hybridation.

## Revendications

**1.** Procédé de stabilisation de l'hybridation d'une première séquence polynucléotidique d'ADN ou d'ARN modifiés ou non, à configuration anomérique $\alpha$ ou $\beta$ avec une seconde séquence cible complémentaire de la première séquence d'ADN ou d'ARN, caractérisé en ce qu'un peptide forme un complexe avec la double hélice d'hybridation que forment lesdites première et seconde séquences nucléotidiques, le peptide prenant une structure en épingle à cheveux dans le grand sillon de ladite double hélice.

2. Procédé selon la revendication 1, caractérisé en ce que le peptide est fixé de manière covalente sur ladite première séquence.

3. Procédé selon la revendication 1 ou 2, caractérisé en ce que l'enchaînement d'aminoacides du peptide comporte :
   - une première succession d'aminoacides qui présentent une interaction un à un respectivement avec une succession de bases de nucléotides de ladite première séquence, le cas échéant, à partir du site de fixation du peptide sur celle-ci,
   - une seconde succession d'un même nombre d'aminoacides que la première succession d'aminoacides, qui présentent une interaction un à un respectivement avec une succession de bases de nucléotides de ladite seconde séquence,
   - lesdites première et seconde successions d'aminoacides étant reliées entre elles par une séquence dite "charnière" constituée notamment par un ou plusieurs acides aminés n'interagissant pas avec les bases desdites première et seconde séquences,

   de telle sorte que :
   - les acides aminés de ladite première succession interagissent de manière coopérative avec les acides aminés de ladite seconde succession,
   - l'interaction coopérative résultante entre le peptide et l'hélice double brin formée par le complexe d'hybridation desdites première et seconde séquences étant telle que le peptide se glisse dans le grand sillon de la double hélice et prenne une structure en épingle à cheveux entre ladite première et ladite seconde séquences nucléotidiques par l'intermédiaire de la séquence dite "charnière".

4. Procédé selon la revendication 1 ou 2, caractérisé en ce que le peptide comporte
   - une première succession d'acides aminés qui présentent une interaction un à un respectivement avec une succession de bases de nucléotides de ladite première séquence,
   - une seconde succession d'acides aminés qui présentent une interaction un à un respectivement avec une succession de bases de nucléotides de ladite seconde séquence,
   - une troisième succession d'acides aminés qui présentent une interaction un à un respectivement avec une succession de bases de nucléotides de ladite première séquence,
   - lesdites première et seconde successions d'acides aminés d'une part, et lesdites seconde et troisième successions d'acides aminés d'autre part, étant reliées à chaque fois par une séquence dite "charnière" identique ou différente constituée notamment par un ou plusieurs acides aminés n'interagissant pas avec les bases desdites première et seconde séquences,
   - les séquences de nucléotides dont les bases interagissent avec ladite seconde succession d'aminoacides constituant la séquence complémentaire de celles dont les bases interagissent avec lesdites première et troisième successions d'acides aminés,

   de telle sorte que :
   - les acides aminés desdites première et troisième successions d'aminoacides interagissent de manière coopérative avec les acides aminés de ladite seconde succession,
   - l'interaction coopérative résultante entre le peptide et l'hélice double brin formée par le complexe d'hybridation desdites première et seconde séquences, étant telle que le peptide se glisse dans le grand sillon de la double hélice et prenne une structure en double épingle à cheveux entre ladite première séquence et ladite seconde séquence nucléotidiques par l'intermédiaire des deux séquences "charnières".

5. Procédé selon l'une des revendications précédentes, caractérisé en ce que les nucléotides de ladite première séquence comportent des bases choisies parmi l'adénine, la guanine, la thymine, la cytosine, l'uracyle, l'inosine, la 2-6 diaminopurine, le 6 amino 8-bromopurine, le 5 bromouracyle, le 5-iodouracyle.

6. Procédé selon l'une des revendications 3 à 5, caractérisé en ce que lorsque la base d'une desdites première ou seconde séquences est une thymine, l'acide aminé correspondant est un acide aminé hydrophobe.

7. Procédé selon la revendication 6, caractériséen ce que l'acide aminé est-choisi parmi la L-phénylalanine, la L-leucine, la L-isoleucine, la L-valine, la L-alanine, la L-méthionine. De préférence, l'acide aminé étant choisi parmi la L-leucine, la L-isoleucine et la L-valine.

**8.** Procédé selon l'une des revendications 3 à 7, caractérisé en ce que lorsque la base est un uracyle, l'acide aminé est un acide aminé amphiphile ou acide.

**9.** Procédé selon la revendication 8, caractérisé en ce que l'acide aminé est choisi par la L-alanine, l'acide L-aspartique, l'acide L-glutamique, la L-sérine, la L-cystéine, la L-thréonine. De préférence, l'acide aminé est choisi parmi la L-alanine, la L-sérine, la L-thréonine.

**10.** Procédé selon l'une des revendications 3 à 9, caractérisé en ce que la base est une cytosine, l'acide aminé étant choisi parmi des acides aminés amphiphiles.

**11.** Procédé selon la revendication 10, caractérisé en ce que l'acide aminé est choisi parmi la L-alanine, la L-sérine, la L-cystéine, la L-thréonine, la L-tyrosine, la L-histidine. De préférence, l'acide aminé étant choisi parmi la L-alanine, la L-sérine, la L-thréonine.

**12.** Procédé selon l'une des revendications 3 à 11, caractérisé en ce que la base est une adénine ou une guanine, l'acide aminé correspondant étant choisi parmi les acides aminés hydrophiles.

**13.** Procédé selon la revendication 12, caractérisé en ce que l'acide aminé est choisi parmi la L-tyrosine, la L-histidine, la L-asparagine, la L-glutamine, la L-lysine, la L-arginine, l'acide L-aspartique, l'acide L-glutamique, la L-sérine, la glycine, la L-thréonine.

**14.** Procédé selon l'une des revendications 3 à 13, caractérisé en ce que lorsque là base est une adénine, l'acide aminé correspondant est choisi parmi les acides aminés très chargés et très hydrophiles, notamment la L-asparagine, la L-glutamine ou la L-lysine et la L-arginine, et lorsque la base est une guanine, l'acide aminé correspondant est choisi parmi les acides aminés petits et hydrophiles, de préférence la L-sérine, la L-cystéine, la L-arginine et la L-lysine.

**15.** Procédé selon l'une des revendications 3 à 13, caractérisé en ce que le peptide est constitué d'un enchaînement d'acides aminés qui ne présentent pas les enchaînements suivants :

| | | |
|---|---|---|
| $AA_x$-Cys-Asp-$AA_z$ | $AA_x$-Cys-Asn-$AA_z$ | $AA_x$-Cys-Glu-AAz |
| AAx-Cys-Gln-AAz | AAx-Asp-Cys-AAz | AAx-Asn-Cys-AAz |
| AAx-Gln-Cys-AAz | AAx-Gln-Cys-AAz | |
| $AA_x$-Trp-Asp-$AA_z$ | $AA_x$-Trp-Gln-AAz | |
| AAx-Asp-Trp-AAz | AAx-Glu-Trp-AAz | |
| AAx-Asp-Gly-AAz | AAx-Asn-Gly-AAz | |
| AAx-GLy-Asp-AAz | AAx-Gly-Asn-AAz | |
| AAx-Gln-Gly-AAz | AAx-Gln-Gly-AAz | |
| AAx-Gly-Gln-AAz | AAx-Gly-Gln-AAz | |
| $AA_x$-Ser-Asp-$AA_z$ | $AA_x$-Ser-Gln-$AA_z$ | |
| $AA_x$-Thr-Asp-$AA_z$ | $AA_x$-Thr-Gln-AAz | |
| AAx-Glu-Thr-AAz | AAx-Asp-Ser-AAz | |
| AAx-Glu-Ser-AAz | AAx-Asp-Thr-AAz. | |

AAx et AAz représentent des acides aminés en amont et en aval, respectant la convention $(NH_2)$-AAx ----- AAz (-COOH).

**16.** Procédé selon l'une des revendications 3 à 15, caractérisé en ce que les acides aminés "charnières" sont choisis parmi la glycine, la L-asparagine et la L-glutamine.

**17.** Conjugué constitué par une séquence polynucléotidique d'ADN ou d'ARN modifiés ou non, à configuration $\alpha$ ou $\beta$, et un peptide dont les acides aminés sont choisis de telle sorte que le peptide prenne une structure en épingle à cheveux ou en double épingle à cheveux dans le grand sillon de la double hélice d'hybridation que forme ladite séquence polynucléotidique avec une séquence d'ADN ou d'ARN complémentaire.

**18.** Conjugué selon la revendication 17, caractérisé en ce que les acides aminés constituant ledit peptide sont choisis en fonction de la nature des nucléotides constituant ladite séquence polynucléotidique compte tenu des principes de correspondance énumérés dans l'une des revendications 3 à 15, ladite séquence polynucléotidique correspondant à ladite première séquence d'ADN ou d'ARN, le peptide étant lié ou non de façon covalente audit polynucléotide.

**19.** Conjugué peptide-polynucléotide utile dans le procédé selon l'une des revendications 2, 3 et 5 à 15, caractérisé en ce qu'il répond à la formule générale I :

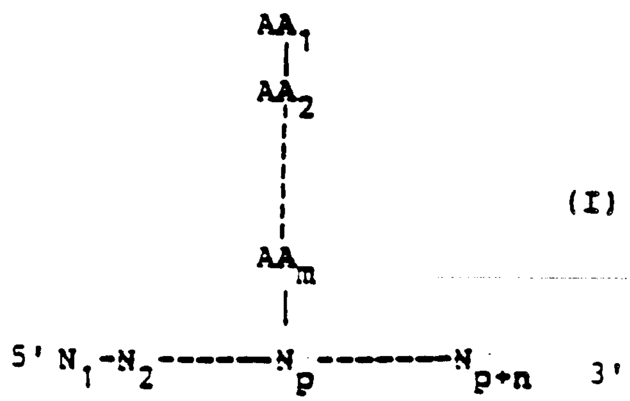

(I)

formule dans laquelle en général :
$100 > p \geqq 1$
$100 > n \geqq 0$
$400 > m \geqq 3$
$AA_1$ ------$AA_m$ représente m acides aminés du peptide, et
$N_1$ -------$N_{p+n}$ représente n + p nucléotides du polynucléotide,
les acides aminés de $AA_1$ ---- $AA_m$ étant choisis conformément aux revendications 2 et 4 à 15.

**20.** Conjugué peptide-polynucléotide utile dans le procédé selon l'une des revendications 3 et 4 à 16, caractérisé en ce qu'il répond à la formule générale (II)

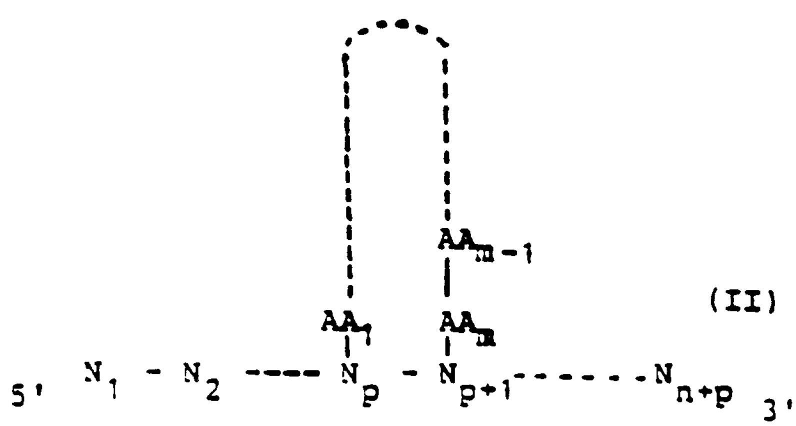

(II)

dans laquelle
- $AA_1$----------$AA_m$ représente m acides aminés du peptide

- $N_1$ ----------$N_{p+n}$ représente ladite première séquence de p + n nucléotide
- $100 > p \geqq 1$
- $100 > n \geqq 1$
- $400 > m \geqq 3$

les acides aminés de $AA_1$ ------ $AA_m$ étant choisis conformément aux revendications 2 et 3 à 9.

**21.** Conjugué selon la revendication 19, caractérisé en ce qu'il répond à la formule générale III

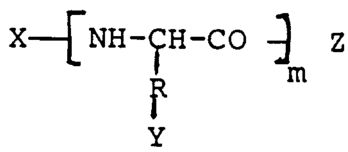

(III)

dans laquelle

m est un nombre entier compris entre 3 et 100,

R est choisi parmi les chaînes d'acides aminés naturels éventuellement modifiés et les chaînes d'acides aminés non naturels éventuellement modifiés,

Z est choisi parmi le radical OH, un polynucléotide, une molécule de protection et une molécule de marquage telle que notamment le dinitrophénol, la biotine, la fluorescéine entre autres,

X est choisi parmi le radical H, un polynucléotide, une molécule de protection et une molécule de marquage telle que notamment le dinitrophénol, la biotine, la fluorescéine entre autres,

Y n'est présent que lorsque R est la chaîne de la lysine, de l'acide aspartique ou de l'acide glutamique, auquel cas Y peut représenter un polynucléotide, une molécule de protection ou une molécule de marquage, notamment le dinitrophénol, la biotine, la fluorescéine, ces éléments étant fixés sur la fonction $NH_2$ ou COOH de R,

l'un seulement de X, Y et Z est un polynucléotide correspondant à ladite première séquence.

**22.** Conjugué selon l'une des revendications 19 à 21, caractérisé en ce que la liaison entre le peptide et le polynucléotide se fait entre une lysine du peptide et une méthyl-cytosine ou cytosine du polynucléotide, le $NH_2$ situé à l'extrémité de la chaîne de la lysine étant fixé sur le carbone en position 4 de la méthyl-cytosine, ou cytosine respectivement.

**23.** Procédé de préparation de conjugués selon la revendication 22, caractérisé en ce que le peptide est lié au polynucléotide en substituant le groupe triazole d'une thymine ou uracile triazolée en position 4 du polynucléotide de départ, par le peptide via son groupe $NH_2$ situé à l'extrémité de la chaîne d'une lysine.

**24.** Composé utile au blocage ou au déblocage d'un gêne de structure ou de régulation situé sur l'ADN d'un organisme quelconque, caractérisé en ce qu'il consiste en un conjugué selon l'une des revendications 19 à 22.

**25.** Composé utile à l'inhibition de la traduction ou de l'épissage d'un ARN messager ou non, caractérisé en ce qu'il consiste en un conjugué selon l'une des revendications 19 à 22.

**26.** A titre de médicament, les conjugués selon l'une des revendications 19 à 22.

**27.** Sonde de diagnostic caractérisée en ce qu'elle est constituée par un conjugué selon l'une des revendications 19 à 22 lié à une molécule de marquage.

## Claims

**1.** Method for stabilizing the hybridization of a first DNA or RNA polynucleotide sequence, modified or not, having an $\alpha$ or $\beta$ anomeric configuration, with a second target sequence complementary to the first DNA or RNA sequence, characterized in that a peptide forms a complex with the double hybridization helix formed by said first and second nucleotide sequences, the peptide adopting a hairpin structure in the major groove of said double helix.

2. Method according to claim 1, characterized in that the peptide is covalently bound to said first sequence.

3. Method according to claims 1 or 2, characterized in that the amino acid chain of the peptide includes:
   - a first succession of amino acids that respectively interacts one to one with a succession of nucleotide bases of said first sequence, if need be, from the binding site of the peptide on the latter,
   - a second succession comprising the same number of amino acids as the first succession of amino acids, that respectively interacts one to one with a succession of nucleotide bases of said second sequence,
   - said first and second successions of amino acids being linked by a so-called "hinge" sequence composed in particular of one or several amino acids which do not interact with the bases of said first and second sequences,

   such that:
   - the amino acids of said first succession interact in a cooperative manner with the amino acids of said second succession,
   - the resulting cooperative interaction between the peptide and the double stranded helix formed by the hybridization complex of said first and second sequences being such that the peptide slides in the major groove of the double helix and assumes a hairpin structure between said first and said second nucleotide sequences by means of the so-called "hinge" region.

4. Method according to claims 1 or 2, characterized in that the peptide includes :
   - a first succession of amino acids that respectively interacts one to one with a succession of nucleotide bases of said first sequence,
   - a second succession of amino acids that respectively interacts one to one with a succession of nucleotide bases of said second sequence,
   - a third succession of amino acids that respectively interacts one to one with a succession of nucleotide bases of said first sequence,
   - said first and second successions of amino acids on the one hand, and said second and third successions of amino acids on the other hand, each being linked by an identical or different so-called "hinge" sequence composed in particular of one or several amino acids which do not interact with the bases of said first and second sequences,
   - the nucleotide sequences whose bases interact with said second succession of amino acids constituting the sequence complementary to those whose bases interact with said first and third successions of amino acids,

   such that:
   - the amino acids of said first and third successions of amino acids interact in a cooperative manner with the amino acids of said second succession,
   - the resulting cooperative interaction between the peptide and the double stranded helix formed by the hybridization complex of said first and second sequences being such that the peptide slides in the major groove of the double helix and assumes a double hairpin structure between said first nucleotide sequence and said second nucleotide sequence by means of the two "hinge" sequences.

5. Method according to one of the preceding claims, characterized in that the nucleotides of said first sequence include bases chosen from among adenine, guanine, thymine, cytosine, uracil, inosine, 2,6-diaminopurine, 6-amino-8-bromopurine, 5-bromouracil, 5-iodouracil.

6. Method according to one of claims 3 to 5, characterized in that when the base of one of said first or second sequences is a thymine, the corresponding amino acid is a hydrophobic amino acid.

7. Method according to claim 6, characterized in that the amino acid is chosen from among L-phenylalanine, L-leucine, L-isoleucine, L-valine, L-alanine, L-methionine, the amino acid preferably being chosen from among L-leucine, L-isoleucine and L-valine.

8. Method according to one of claims 3 to 7, characterized in that when the base is a uracil, the amino acid is an amphipathic or acidic amino acid.

**9.** Method according to claim 8, characterized in that the amino acid is chosen from among L-alanine, L-aspartic acid, L-glutamic acid, L-serine, L-cysteine, L-threonine, the amino acid preferably being chosen from among L-alanine, L-serine, L-threonine.

**10.** Method according to one of claims 3 to 9, characterized in that when the base is a cytosine, the amino acid is chosen from among the amphipathic amino acids.

**11.** Method according to claim 10, characterized in that the amino acid is chosen from among L-alanine, L-serine, L-cysteine, L-threonine, L-tyrosine, L-histidine, the amino acid preferably being chosen from among L-alanine, L-serine, L-threonine.

**12.** Method according to one of claims 3 to 11, characterized in that when the base is an adenine or a guanine, the corresponding amino acid is chosen from among the hydrophilic amino acids.

**13.** Method according to claim 12, characterized in that the amino acid is chosen from among L-tyrosine, L-histidine, L-asparagine, L-glutamine, L-lysine, L-arginine, L-aspartic acid, L-glutamic acid, L-serine, glycine, L-threonine.

**14.** Method according to one of claims 3 to 13, characterized in that when the base is an adenine, the corresponding amino acid is chosen from among the highly charged and very hydrophilic amino acids, particularly L-asparagine, L-glutamine or L-lysine and L-arginine, and when the base is a guanine, the corresponding amino acid is chosen from among the small, hydrophilic amino acids, preferably L-serine, L-cysteine, L-arginine and L-lysine.

**15.** Method according to one of claims 3 to 13, characterized in that the peptide is composed of a chain of amino acids in which the following sequences do not occur:

| | | |
|---|---|---|
| $AA_x$ - Cys - Asp - $AA_z$ | $AA_x$ - Cys - Asn - $AA_z$ | $AA_x$ - Cys - Glu - $AA_z$ |
| $AA_x$ - Cys - Gln - $AA_z$ | $AA_x$ - Asp - Cys - $AA_z$ | $AA_x$ - Asn - Cys - $AA_z$ |
| $AA_x$ - Gln - Cys - $AA_z$ | $AA_x$ - Gln - Cys - $AA_z$ | |
| $AA_x$ - Trp - Asp - $AA_z$ | $AA_x$ - Trp - Gln - $AA_z$ | |
| $AA_x$ - Asp - Trp- $AA_z$ | $AA_x$ - Glu - Trp - $AA_z$ | |
| $AA_x$ - Asp - Gly- $AA_z$ | $AA_x$ - Asn - Gly - $AA_z$ | |
| $AA_x$ - Gly - Asp - $AA_z$ | $AA_x$ - Gly - Asn - $AA_z$ | |
| $AA_x$ - Gln - Gly - $AA_z$ | $AA_x$ - Gln - Gly - $AA_z$ | |
| $AA_x$ - Gly - Gln - $AA_z$ | $AA_x$ - Gly - Gln - $AA_z$ | |
| $AA_x$ - Ser - Asp - $AA_z$ | $AA_x$ - Ser - Gln - $AA_z$ | |
| $AA_x$ - Thr - Asp - $AA_z$ | $AA_x$ - Thr - Gln - $AA_z$ | |
| $AA_x$ - Glu - Thr - $AA_z$ | $AA_x$ - Asp - Ser - $AA_z$ | |
| $AA_x$ - Glu - Ser - $AA_z$ | $AA_x$ - Asp -Thr - $AA_z$. | |

$AA_x$ and $AA_z$ represent the upstream and downstream amino acids, in accordance with the convention $(NH_2)$ - $AA_x$------$AA_z$ (-COOH).

**16.** Method according to one of claims 3 to 15, characterized in that the "hinge" amino acids are chosen from among glycine, L-asparagine and L-glutamine.

**17.** Conjugate composed of a DNA or RNA polynucleotide sequence, modified or not, with an $\alpha$ or $\beta$ configuration, and a peptide whose amino acids are chosen so that the peptide adopts a hairpin or double hairpin structure in the major groove of the double hybridization helix formed by said polynucleotide sequence with a complementary DNA or RNA sequence.

**18.** Conjugate according to claim 17, characterized in that the amino acids constituting said peptide are chosen as a function of the nature of the nucleotides constituting said polynucleotide sequence, taking into account the principles of correspondence listed in one of claims 3 to 15, said polynucleotide sequence corresponding to said first DNA or RNA sequence, the peptide being covalently bound or not to said polynucleotide.

**19.** Peptide-polynucleotide conjugate useful in the method according to one of the claims 2, 3 and 5 to 15, characterized in that it possesses the general formula I:

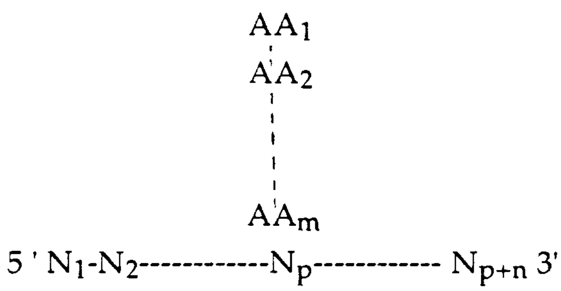

(I)

a formula in which, in general:
$100>p\geqq 1$
$100>n\geqq 0$
$400>m\geqq 3$
$AA_1$----------$AA_m$ represent m amino acids of the peptide, and
$N_1$-----------$N_{p+n}$ represent n + p nucleotides of the polynucleotide,
the amino acids $AA_1$----------$AA_m$ being chosen in accordance with claims 2 and 4 to 15.

**20.** Peptide-polynucleotide conjugate useful in the method according to one of the claims 3 and 4 to 16, characterized in that it possesses the general formula II:

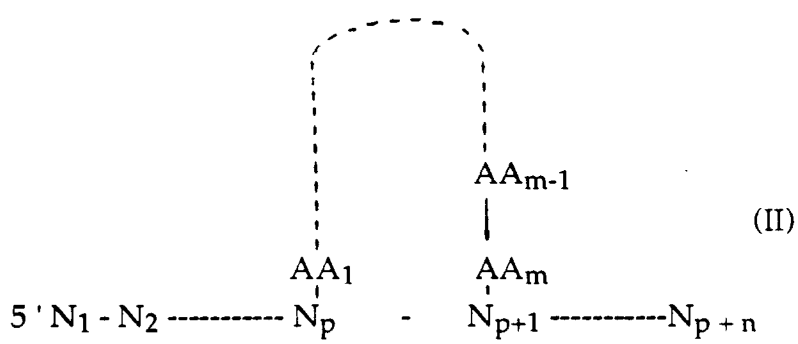

(II)

in which
- $AA_1$----------$AA_m$ represent m amino acids of the peptide
- $N_1$-----------$N_{p+n}$ represent said first sequence of p + n nucleotides
- $100>p\geqq 1$
- $100>n\geqq 1$
- $400>m\geqq 3$

the amino acids $AA_1$----------$AA_m$ being chosen in accordance with claims 2 and 3 to 9.

**21.** Conjugate according to claim 19, characterized in that it possesses the general formula III

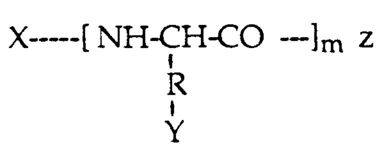

(III)

in which

- m is a whole number between 3 and 100,
- R is chosen from chains of natural amino acids, possibly modified, and chains of non natural amino acids, possibly modified,
- Z is chosen from among the OH group, a polynucleotide, a protective group and a molecular label such as in particular dinitrophenol, biotin, fluorescein, among others,
- X is chosen from among the group H, a polynucleotide, a protective group or a molecular label such as in particular dinitrophenol, biotin, fluorescein, among others,
- Y is present only when R is the chain of lysine, aspartic acid or glutamic acid, in which case Y may represent a polynucleotide, a protective group or a molecular label, in particular dinitrophenol, biotin, fluorescein, these elements being bound to the $NH_2$ or COOH group of R,

only one of X, Y and Z being a polynucleotide corresponding to said first sequence.

**22.** Conjugate according to one of claims 19 to 21, characterized in that the bond between the peptide and the polynucleotide is between a lysine of the peptide and a methylcytosine or a cytosine of the polynucleotide, the $NH_2$ located at the extremity of the lysine chain being bound to the carbon in position 4 of the methylcytosine or cytosine, respectively.

**23.** Method of preparation of conjugates according to claim 22, characterized in that the peptide is bound to the polynucleotide by substituting the triazole group of a uracil or thymine triazolated in position 4 of the starting polynucleotide, by the peptide via its $NH_2$ group located at the extremity of a lysine chain.

**24.** Compound useful for the blocking or deblocking of a structural or regulatory gene in the DNA of any organism, characterized in that it consists of a conjugate according to one of claims 19 to 22.

**25.** Compound useful for inhibiting or not the translation or splicing of messenger RNA, characterized in that it consists of a conjugate according to one of claims 19 to 22.

**26.** For pharmaceutical purposes, the conjugates according to one of claims 19 to 22.

**27.** Diagnostic probe characterized in that it consists of a conjugate according to one of claims 19 to 22 bound to a molecular label.

## Patentansprüche

**1.** Verfahren zur Stabilisierung der Hybridisierung einer ersten Polynucleotid-Sequenz von modifizierter oder nichtmodifizierter DNA oder RNA mit anomerer $\alpha$- oder $\beta$-Konfiguration mit einer zweiten Zielsequenz, die komplementär zur ersten DNA- oder RNA-Sequenz ist, dadurch gekennzeichnet, daß ein Peptid einen Komplex bildet mit der Hybridisierungs-Doppelhelix, welche die genannten ersten und zweiten Nucleotid-Sequenzen bilden, wobei das Peptid in der großen Furche (Rille) der genannten Doppelhelix eine Haarspangen-Struktur annimmt.

**2.** Verfahren nach Anspruch 1, dadurch gekennzeichnet, daß das Peptid kovalent an die genannte erste Sequenz gebunden wird.

**3.** Verfahren nach Anspruch 1 oder 2, dadurch gekennzeichnet, daß die Verknüpfung (Verkettung) der Aminosäuren des Peptids umfaßt:

- eine erste Folge von Aminosäuren, die jeweils einzeln eine Wechselwirkung mit einer Folge von Nucleotid-Basen der genannten ersten Sequenz aufweisen, gegebenenfalls ab der Bindungsstelle des Peptids auf derselben,
- eine zweite Folge einer gleichen Anzahl von Aminosäuren wie die erste Folge von Aminosäuren, die einzeln jeweils eine Wechselwirkung mit einer Folge von Nucleotid-Basen der genannten zweiten Sequenz aufweisen,
- wobei die genannten ersten und zweiten Folgen von Aminosäuren durch eine als "Scharnier-(Gelenk)" bezeichnete Sequenz miteinander verbunden sind, die insbesondere besteht aus einer oder mehreren Aminosäuren, die nicht in Wechselwirkung treten mit den Basen der genannten ersten und zweiten Sequenzen, in der Weise, daß:

- die Aminosäuren der genannten ersten Folge kooperativ in Wechselwirkung treten mit den Aminosäuren der genannten zweiten Folge,
- wobei die resultierende kooperative Wechselwirkung zwischen dem Peptid und der Doppelstrang-Helix, die durch den Hybridisierungskomplex der genannten ersten und zweiten Sequenzen gebildet wird, so ist, daß das Peptid in der großen Furche (Rille) der Doppelhelix gleitet und zwischen der genannen ersten Nucleotid-Sequenz und der genannten zweiten Nucleotid-Sequenz mittels der als "Scharnier(Gelenk)" bezeichneten Sequenz eine Haarspangen-Struktur annimmt.

**4.** Verfahren nach Anspruch 1 oder 2, dadurch gekennzeichnet, daß das Peptid umfaßt
- eine erste Folge von Aminosäuren, die einzeln jeweils eine Wechselwirkung mit einer Folge von Nucleotid-Basen der genannten ersten Sequenz aufweisen,
- eine zweite Folge von Aminosäuren, die einzeln jeweils eine Wechselwirkung mit einer Folge von Nucleotid-Basen der genannten zweiten Sequenz aufweisen,
- eine dritte Folge von Aminosäuren, die einzeln jeweils eine Wechselwirkung mit einer Folge von Nucleotid-Basen der genannten ersten Sequenz aufweisen,
- wobei die genannten ersten und zweiten Folgen von Aminosäuren einerseits und die genannten zweiten und dritten Folgen von Aminosäuren andererseits jedesmal durch eine als "Scharnier-(Gelenk)" bezeichnete, identische oder unterschiedliche Sequenz, die insbesondere aus einer oder mehreren Aminosäuren, die nicht mit den Basen der genannten ersten und zweiten Sequenzen in Wechselwirkung treten, verbunden sind,
- die Nucleotid-Sequenzen, deren Basen mit der genannten zweiten Folge von Aminosäuren in Wechselwirkung treten, welche die Sequenz darstellen, die komplementär zu denjenigen ist, deren Basen mit den genannten ersten und dritten Folgen von Aminosäuren in Wechselwirkung treten, in der Weise, daß
- die Aminosäuren der genannten ersten und dritten Folgen von Aminosäuren kooperativ in Wechselwirkung treten mit den Aminosäuren der genannten zweiten Folge,
- wobei die resultierende kooperative Wechselwirkung zwischen dem Peptid und der Doppelstrang-Helix, die durch den Hybridisierungskomplex der genannten ersten und zweiten Sequenzen gebildet worden ist, so ist, daß das Peptid in der großen Furche (Rille) der Doppelhelix gleitet und zwischen der genannten ersten Nucleotid-Sequenz und der genannten zweiten Nucleotid-Sequenz mittels der beiden "Scharnier(Gelenk)"-Sequenzen eine Doppel-Haarspangen-Struktur annehmen.

**5.** Verfahren nach einem der vorhergehenden Ansprüche, dadurch gekennzeichnet, daß die Nucleotide der genannten ersten Sequenz Basen aus der Gruppe Adenin, Guanin, Thymin, Cytosin, Uracil, Inosin, 2,6-Diaminopurin, 6-Amino-8-bromopurin, 5-Bromouracil und 5-Jodouracil aufweisen.

**6.** Verfahren nach einem der Ansprüche 3 bis 5, dadurch gekennzeichnet, daß dann, wenn die Base einer der genannten ersten und zweiten Sequenzen ein Thymin ist, die entsprechende Aminosäure eine hydrophobe Aminosäure ist.

**7.** Verfahren nach Anspruch 6, dadurch gekennzeichnet, daß die Aminosäure ausgewählt wird aus der Gruppe L-Phenylalanin, L-Leucin, L-Isoleucin, L-Valin, L-Alanin und L-Methionin, wobei die Aminosäure vorzugsweise ausgewählt wird aus der Gruppe L-Leucin, L-Isoleucin und L-Valin.

**8.** Verfahren nach einem der Ansprüche 3 bis 7, dadurch gekennzeichnet, daß dann, wenn die Base ein Uracil ist, die Aminosäure eine amphiphile oder saure Aminosäure ist.

**9.** Verfahren nach Anspruch 8, dadurch gekennzeichnet, daß die Aminosäure ausgewählt wird aus der Gruppe L-Alanin, L-Asparaginsäure, L-Glutaminsäure, L-Serin, L-Cystein und L-Threonin, wobei die Aminosäure vorzugsweise ausgewählt wird aus der Gruppe L-Alanin, L-Serin und L-Threonin.

**10.** Verfahren nach einem der Ansprüche 3 bis 9, dadurch gekennzeichnet, daß die Base ein Cytosin ist und daß die Aminosäure ausgewählt wird aus amphiphilen Aminosäuren.

**11.** Verfahren nach Anspruch 10, dadurch gekennzeichnet, daß die Aminosäure ausgewählt wird aus der Gruppe L-Alanin, L-Serin, L-Cystein, L-Threonin, L-Thyrosin und L-Histidin, wobei die Aminosäure vorzugsweise ausgewählt wird aus der Gruppe L-Alanin, L-Serin und L-Threonin.

**12.** Verfahren nach einem der Ansprüche 3 bis 11, dadurch gekennzeichnet, daß die Base ein Adenin oder ein Guanin ist, wobei die entsprechende Aminosäure ausgewählt wird aus den hydrophilen Aminosäuren.

**13.** Verfahren nach Anspruch 12, dadurch gekennzeichnet, daß die Aminosäure ausgewählt wird aus der Gruppe L-Thyrosin, L-Histidin, L-Asparagin, L-Glutamin, L-Lysin, L-Arginin, L-Asparaginsäure, L-Glutaminsäure, L-Serin, L-Glycin und L-Threonin.

**14.** Verfahren nach einem der Ansprüche 3 bis 13, dadurch gekennzeichnet, daß dann, wenn die Base ein Adenin ist, die entsprechende Aminosäure ausgewählt wird aus den sehr schweren (geladenen) und sehr hydrophilen Aminosäuren, insbesondere aus der Gruppe L-Asparagin, L-Glutamin oder L-Lysin und L-Arginin, und daß dann, wenn die Base ein Guanin ist, die entsprechende Aminosäure ausgewählt wird aus den kleinen und hydrophilen Aminosäuren, vorzugsweise aus der Gruppe L-Serin, L-Cystein, L-Arginin und L-Lysin.

**15.** Verfahren nach einem der Ansprüche 3 bis 13, dadurch gekennzeichnet, daß das Peptid besteht aus einer Verknüpfung (Verkettung) von Aminosäuren, welche die folgenden Verknüpfungen (Verkettungen) nicht aufweisen:

| | | |
|---|---|---|
| $AA_x$-Cys-Asp-$AA_z$ | $AA_x$-Cys-Asn-$AA_z$ | $AA_x$-Cys-Glu-AAz |
| AAx-Cys-Gln-AAz | AAx-Asp-Cys-AAz | AAx-Asn-Cys-AAz |
| AAx-Gln-Cys-AAz | AAx-Gln-Cys-AAz | |
| $AA_x$-Trp-Asp-$AA_z$ | $AA_x$-Trp-Gln-AAz | |
| AAx-Asp-Trp-AAz | AAx-Glu-Trp-AAz | |
| AAx-Asp-Gly-AAz | AAx-Asn-Gly-AAz | |
| AAx-GLy-Asp-AAz | AAx-Gly-Asn-AAz | |
| AAx-Gln-Gly-AAz | AAx-Gln-Gly-AAz | |
| AAx-Gly-Gln-AAz | AAx-Gly-Gln-AAz | |
| $AA_x$-Ser-Asp-$AA_z$ | $AA_x$-Ser-Gln-$AA_z$ | |
| $AA_x$-Thr-Asp-$AA_z$ | $AA_x$-Thr-Gln-AAz | |
| AAx-Glu-Thr-AAz | AAx-Asp-Ser-AAz | |
| AAx-Glu-Ser-AAz | AAx-Asp-Thr-AAz. | |

wobei AAs und AAz entsprechend der Konvention
($NH_2$)-AAx ----- AAz (-COOH) stromaufwärts und stromabwärts Aminosäuren darstellen.

**16.** Verfahren nach einem der Ansprüche 3 bis 15, dadurch gekennzeichnet, daß die "Scharnier(Gelenk)"-Aminosäuren ausgewählt werden aus der Gruppe Glycin, L-Asparagin und L-Glutamin.

**17.** Konjugat, das besteht aus einer Polynucleotid-Sequenz von modifizierter oder nicht-modifizierter DNA oder RNA mit $\alpha$- oder $\beta$-Konfiguration und einem Peptid, dessen Aminosäuren so ausgewählt werden, daß das Peptid in der großen Furche (Rille) der Hybridisierungs-Doppelhelix, welche die genannte Polynucleotid-Sequenz mit einer komplementären DNA- oder RNA-Sequenz bildet, eine Haarspangen- oder Doppel-Haarspangen-Struktur annimmt.

**18.** Konjugat nach Anspruch 17, dadurch gekennzeichnet, daß die Aminosäuren, die das genannte Peptid aufbauen, ausgewählt werden als Funktion der Art der Nucleotide, welche die genannte Polynucleotidsequenz aufbauen, unter Berücksichtigung der in einem der Ansprüche 3 bis 15 aufgezählten Korrespondenz-Prinzipien, wobei in der genannten Polynucleotidsequenz, die der genannten ersten DNA- oder RNA-Sequenz entspricht, das Peptid kovalent oder nicht-kovalent an das genannte Polynucleotid gebunden ist.

**19.** Peptid-Polynucleotid-Konjugat, das verwendbar ist in dem Verfahren nach einem der Ansprüche 2, 3 und 5 bis 15, dadurch gekennzeichnet, daß es der allgemeinen Formel (I) entspricht:

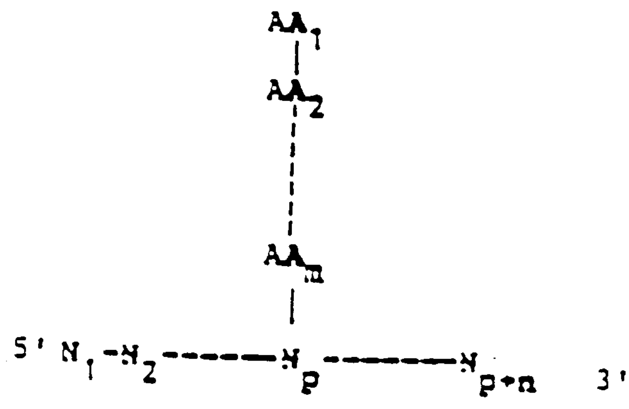

(I)

in der im allgemeinen bedeuten:

$100 > p \geqq 1$

$100 > n \geqq 0$

$400 > m \geqq 3$

$AA_1$ ------$AA_m$ m Aminosäuren des Peptids und

$N_1$ -------$N_{p+n\ n+p}$ Nucleotide des Polynucleotids,

wobei die Aminosäuren $AA_1$ ---- $AA_m$ entsprechend den Ansprüchen 2 und 4 bis 15 ausgewählt worden sind.

**20.** Peptid-Polynucleotid-Konjugat, das in dem Verfahren nach einem der Ansprüche 3 und 4 bis 16 verwendbar ist, dadurch gekennzeichnet, daß es der allgemeinen Formel (II) entspricht:

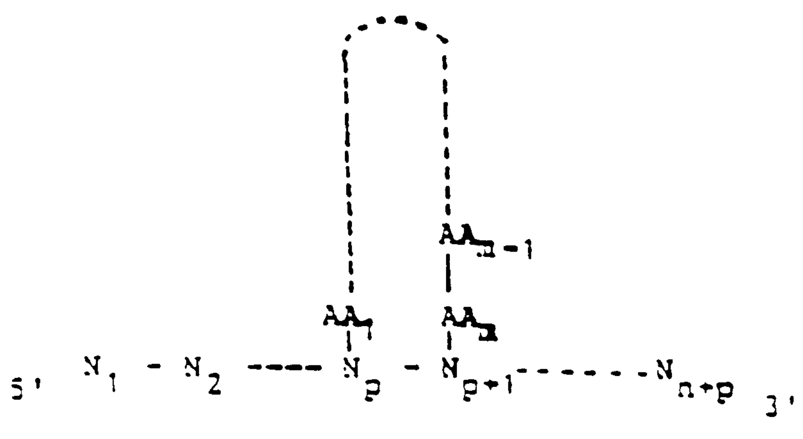

(II)

worin bedeuten:

- $AA_1$----------$AA_m$ m Aminosäuren des Peptids,
- $N_1$ ----------$N_{p+n}$ die genannte erste Sequenz des p + n-Nucleotids
- $100 > p \geqq 1$
- $100 > n \geqq 1$
- $400 > m \geqq 3$

wobei die Aminosäuren $AA_1$ ------ $AA_m$ entsprechend den Ansprüchen 2 und 3 bis 9 ausgewählt worden sind.

**21.** Konjugat nach Anspruch 19, dadurch gekennzeichnet, daß es der allgemeinen Formel (III) entspricht

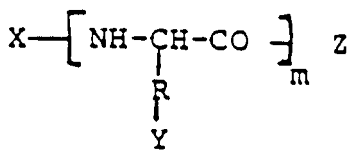

(III)

worin

- m steht für eine ganze Zahl zwischen 3 und 100,
- R ausgewählt wird aus den Ketten von gegebenenfalls modifizierten natürlichen Aminosäuren und den Ketten von gegebenenfalls modifizierten nicht-natürlichen Aminosäuren,
- Z ausgewählt wird aus der Gruppe, die besteht aus einem OH-Rest, einem Polynucleotid, einem Schutzmolekül und einem Markierungsmolekül, wie insbesondere unter anderen Dinitrophenol, Biotin und Fluorescein,
- X ausgewählt wird aus der Gruppe, die besteht aus dem H-Rest, einem Polynucleotid, einem Schutzmolekül und einem Markierungsmolekül, wie insbesondere unter anderen Dinitrophenol, Biotin und Fluorescein,
- Y nur dann vorhanden ist, wenn R die Kette von Lysin, Asparaginsäure oder Glutaminsäure darstellt, wobei in diesem Falle Y ein Polynucleotid, ein Schutzmolekül oder ein Markierungsmolekül, insbesondere Dinitrophenol, Biotin und Fluorescein, darstellen kann, wobei diese Elemente an die $NH_2$- oder COOH-Funktion von R gebunden sind,

wobei nur einer der Reste X, Y und Z ein der genannten ersten Sequenz entsprechendes Polynucleotid ist.

**22.** Konjugat nach einem der Ansprüche 19 bis 21, dadurch gekennzeichnet, daß die Bindung zwischen dem Peptid und dem Polynucleotid erfolgt zwischen einem Lysin des Peptids und einem Methylcytosin oder Cytosin des Polynucleotids, wobei die am Ende der Kette des Lysins angeordnete $NH_2$-Gruppe über das Kohlenstoffatom in der 4-position des Methylcytosins bzw. des Cytosins gebunden ist.

**23.** Verfahren zur Herstellung von Konjugaten nach Anspruch 22, dadurch gekennzeichnet, daß das Peptid an das Polynucleotid gebunden wird, indem man die Triazolgruppe eines triazolierten Thymins oder Uracils in der 4-Position des Ausgangs-Polynucleotids durch das Peptid über seine am Ende der Kette eines Lysins angeordnete $NH_2$-Gruppe substituiert.

**24.** Verbindung, die verwendbar ist zur Blockierung oder Deblockierung eines Struktur- oder Regulations-Gens, das auf der DNA eines beliebigen Organismus angeordnet ist, dadurch gekennzeichnet, daß sie besteht aus einem Konjugat nach einem der Ansprüche 19 bis 22.

**25.** Verbindung, die verwendbar ist zur Inhibierung der Translation oder Spleißung einer Messenger-RNA oder Nicht-Messenger-RNA, dadurch gekennzeichnet, daß sie besteht aus einem Konjugat nach einem der Ansprüche 19 bis 22.

**26.** Die Konjugate nach einem der Ansprüche 19 bis 22 als Arzneimittel.

**27.** Diagnostische Sonde, dadurch gekennzeichnet, daß sie besteht aus einem Konjugat nach einem der Ansprüche 19 bis 22, das an ein Markierungsmolekül gebunden ist.

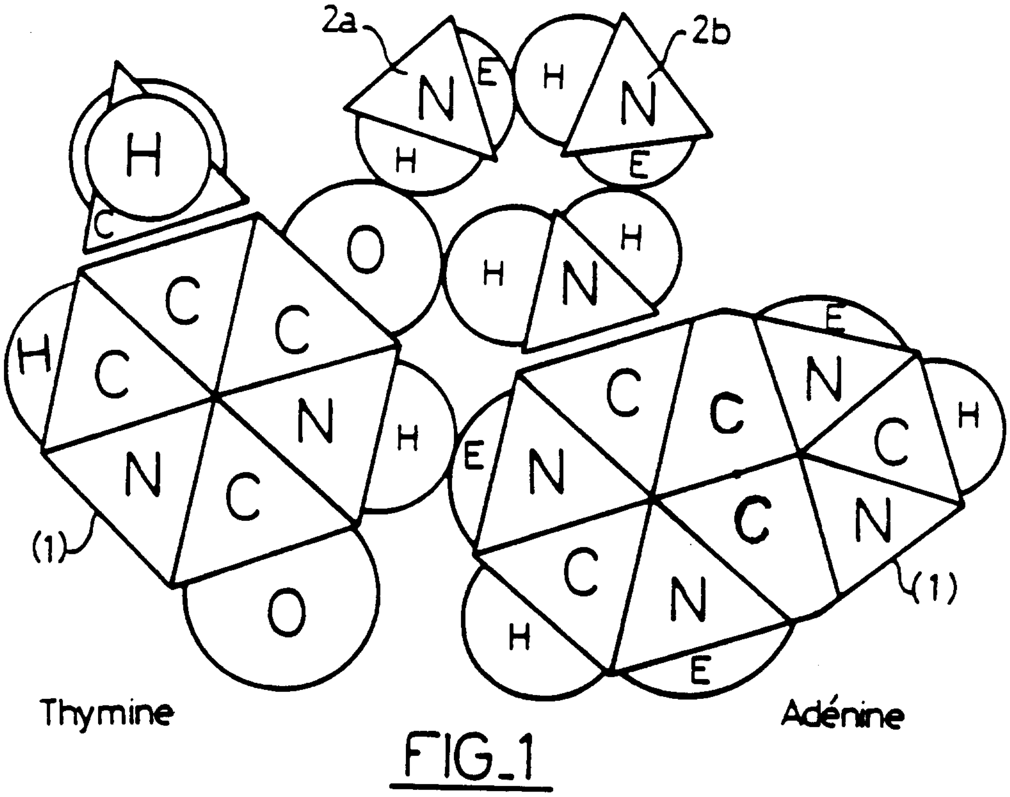

Thymine Adénine

FIG_1

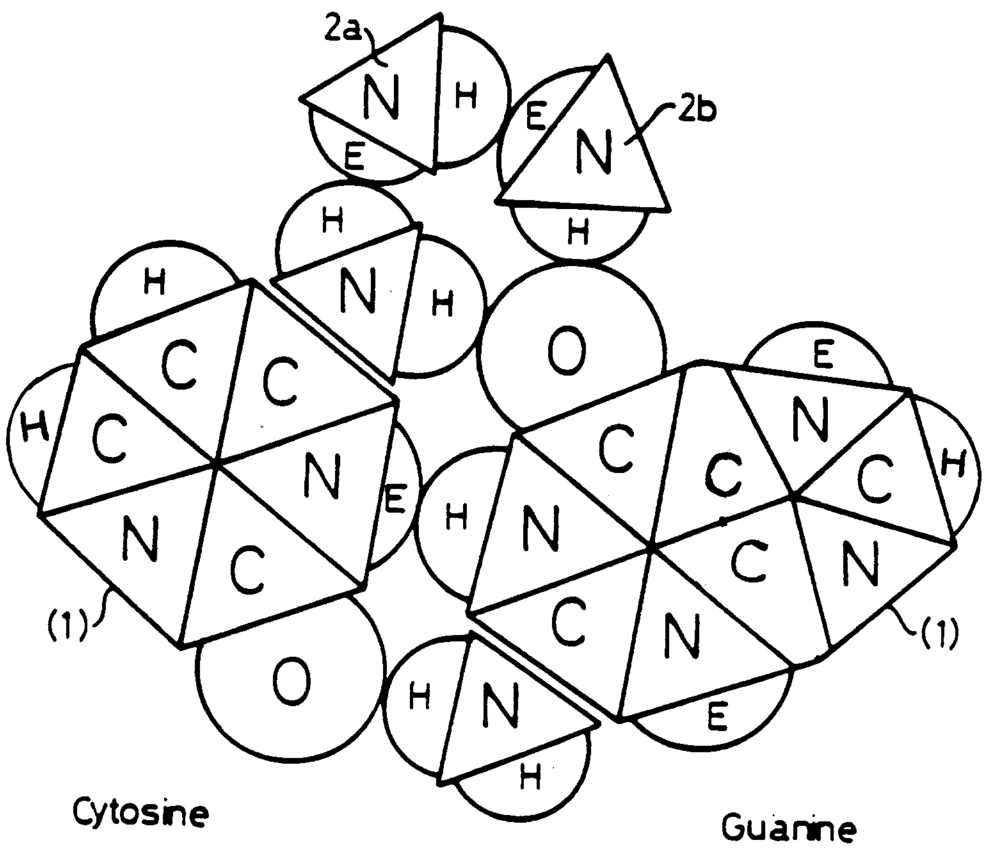

Cytosine Guanine

FIG_2

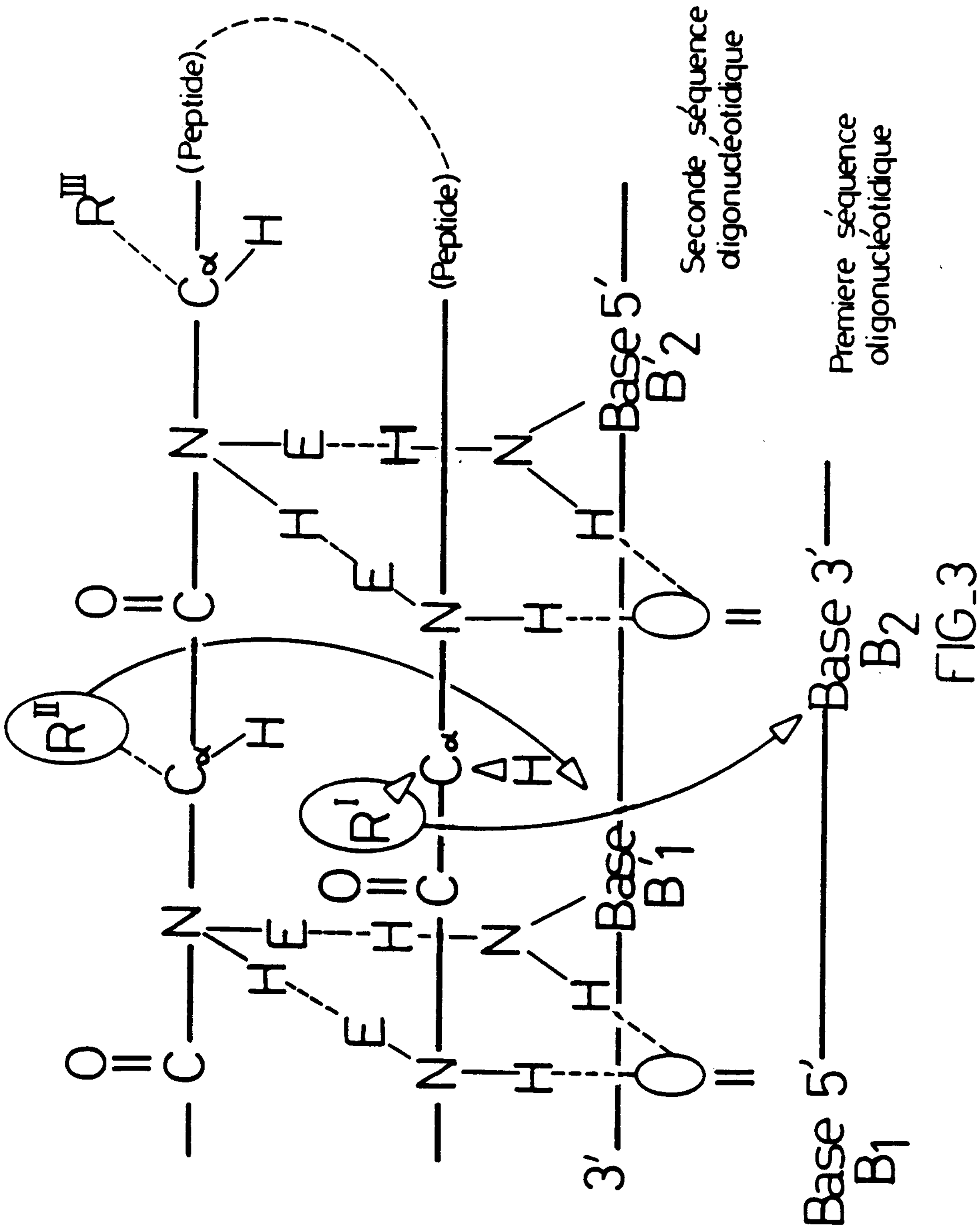
Seconde séquence oligonucléotidique
Premiere séquence oligonucléotidique

FIG_3